# EUROPEAN PATENT APPLICATION

(11) **EP 2 711 366 A1**
(43) Date of publication of application: **26.03.2014**
(21) Application number: 12306133.5
(22) Date of filing: 19.09.2012
(51) Int. Cl.: C07D 407/04, C07D 413/04, C09B 57/00, A61K 31/422, A61K 31/4425, A61K 31/4439, A61K 31/4184, A61P 35/00

(54) **Cationic triphenylamine derivatives activable by visible and Infra Red light for inducing and imaging apoptosis in cancer cells**

(71) Applicant: Institut Curie, 75005 Paris (FR); Centre National de la Recherche Scientifique - CNRS -, 75794 Paris Cedex 16 (FR); ECOLE NORMALE SUPERIEURE DE CACHAN, F-94230 Cachan (FR)
(72) Inventor: The designation of the inventor has not yet been filed
(74) Representative: Cabinet Plasseraud

(57) **Abstract**

The present invention relates to cationic triphenylamines derivatives compounds having a common structure presenting the following general formula (A) activable by visible and infra-red light for inducing and imaging apoptosis in cancer cells 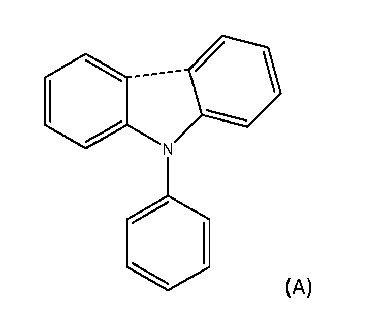 where
---- is an optional covalent bond

## Description

### TECHNICAL FIELD

The present invention relates to cationic triphenylamine derivates and to their use in dynamic phototherapy (PDT).

### BACKGROUND OF THE INVENTION

Over the last thirty years, photodynamic therapy (PDT hereafter) has known considerable development in a wide range of medical applications.

PDT is a treatment that uses a photosensitizer or photosensitizing agent, and a particular type of light (in most cases, in the visible region of the spectrum). When said photosensitizers are exposed to a specific wavelength of light, it causes escape of reactive oxygen species (ROS) that induces apoptosis of cancer cells.

Currently, two main families of compounds are used in dynamic phototherapy: porphyrin and chlorine.

Among the plurality of porphyrin and chlorine derivatives, Photofrin^{®} was the first PDT compound approved for clinical use. However, it has several drawbacks including low molar absorption (extinction coefficient of 3200 M⁻¹ cm⁻¹ at 630 nm) and prolonged skin photosensitivity due to a low stringency in terms of light dose response (patients are prohibited from going out into direct sunlight for minimally 1 month (usually 4-8 weeks) after administration).

PDT compounds of second generation, including well-known chlorine compounds, are characterized by a better absorption of light but they do not allow to overcome the problem of skin photosensitivity (patients are prohibited from going out into direct sunlight (protection of the eyes and skin) for 1 week up to 6 weeks after administration of chlorine derivatives (Moore et al. 2009 Nature Clinical Practice 6, 18-30*)).*

Besides the porphyrin and chlorine family compounds, Rose Bengal acetate (RBA) (Panzarini et al. 2011 Cell Death & Disease 2, e16*)* and two cationic compounds, namely methylene blue (MB) and crystal violet (CV) *(*Oliveira et al. 2011 Free Radical Biology & Medicine 51, 824-33*),* have been described to be potent photosensitizers for PDT, most likely targeting perinuclear region, lysosomes and mitochondria, respectively. However, they are highly sensitive to light illumination (total fluence or light dose: 1.7 J/cm² for MB and CV with 532 or 665-nm line laser sources, 1.6 J/cm² for RBA with 532 or 665-nm line laser sources).

Moreover, the fluorescent properties of the above PDT compounds described above are low. Therefore, high concentrations of these compounds have to be used in order to image their fates in the cell context during the apoptosis phenomenon.

There are two different possible ways to achieve excitation of compounds in PDT: one-photon excitation process and two-photon excitation process.

The development of two-photon PDT compounds is of particular interest for two main reasons:
(i) the excitation wavelength in the near-infra red region of the spectrum offers deeper penetration in the tissues. The high penetration using two-photon excitation is possible because above 780 nm, the IR light is not or much less attenuated by absorption and light scattering. However, above 930-950 nm, the IR absorption of water significantly occurs and accounts for heat release. This intrinsically defines a therapeutic window for in vivo two-photon applications: typically 780-950 nm.
(ii) The two-photon excitation process allows to intrinsically determine the area of excitation in a very specific manner as the excitation can be pin-pointed to a small excitation volume (typically below 1 femtoliter) at the laser focus, providing a highly and accurate targeted treatment.

The current available photosensitizers display absorption peaks compatible with photo activation in the one-photon therapeutic window (i.e. in the red part of the visible spectrum: absorption peaks are centered around 630 nm for porphyrins, 652 nm for mTHPC (chlorine derivative) and 670 nm for phthalocyanine *(*Yow et al. 2000 Cancer Letters 157, 123-31 *and references herein).* Nevertheless, their two-photon absorption properties are strongly limited.

The efficiency by which a compound undergoes two photon absorption is related to its cross section (in Göppert Mayer units where 1 GM = 10⁻⁵⁰ cm⁴ s photon⁻¹ molecule⁻¹). Most of the current available PDT compounds are not compatible with two-photon PDT approaches. Indeed, their two-photon absorption cross sections are much below 100 GM, which is too small to be clinically useful. For instance, the commercial photosensitiser Visudyne® is characterized by a low two-photon cross section of 46 GM at 920 nm *(*Dahlstedt et al. 2009 Org. Biomol. Chem. 7, 897-904*)*. Photofrin® and chlorin e6 (Radachlorin®) have also a very low two-photon cross-section (10 and 5 GM at 800 and 900 nm, respectively, for Photofrin® and 29 GM at 800 nm for chlorin e6) *(*Karotki et al. 2006 Photochem. Photobiol. 82,443-52*).* Therefore, their use under two-photon excitation conditions means that these compounds require a high irradiation intensity (typically 6300 J/cm²) which could account for photodamage during treatment *(*Karotki et al. 2006 Photochem. Photobiol. 82, 443-52*)*.

Up to now, two PDT compounds have been published to be compatible with two-photon absorption without encapsulation strategy (it means that these compounds penetrate by themselves in the targeted cells without vectorization): porphyrin dimers *(*Dahlstedt et al. 2009 Org. Biomol. Chem. 7, 897-904*)* and a porphyrin derivative with two covalently linked bis(diphenylamino)distyrylbenzene groups *(*Starkey et al. 2008 Clin. Cancer Res. 14, 6564). However, these compounds present many drawbaks.

Indeed, firstly, these compounds remain highly sensitive to natural light as they display their one-photon absorption band around 657 nm and 700 nm *(*Dahlstedt et al. 2009 Org. Biomol. Chem. 7, 897-904; Starkey et al. 2008 Clin. Cancer Res. 14, 6564), with little stringency regarding the light dose (fluence) response (in the 2-11 J/cm² range).

Secondly, the separation of one- and two-photon effects is not easy since the one-photon absorption band used in PDT (657 or 700 nm) is close to the near IR. Thus, in this case, one-photon excitation in the long wavelength tail of the one-photon absorption spectrum can be misinterpreted as a 2-photon excitation process.

Thirdly, regarding porphyrin dimers, the compounds do not localise in mitochondria *(*Kuimova et al. 2009 Org. Biomol. Chem. 7, 889-96) which is widely described as to be the most optimal localization for efficient light-induced apoptosis.

Furthermore, the development of two-photon PDT compounds is of particular interest, in the context where optical fibers capable of carrying high-peak intensity laser pulses are currently in development. Indeed, one-photon optical fibers are widely used. Confocal fiber bundles offer a relatively poor transverse resolution of 3 to 5 µm and may not be suitable for delivering short pulses for multiphoton imaging. Recently, several research groups have demonstrated promising endoscope technologies based on fiber optic bundles and gradient-index (GRIN) lenses. GRIN endoscopes have been demonstrated to have cellular resolution for one-photon wide-field and two-photon fluorescence imaging *(*Gu M., Bao HC and Li JL. 2010 J. Biomed. Optics 15, 050502*;* Pilhan Kim et al. 2008 Journal of Biomed. Optics 13, 010501*;* Christoph J. Engelbrecht, et al. 2008 Optics Express Vol . 16(8), pp. 5556-64*;* Juergen C. Jung and Mark J. Schnitzer 2003 Optics Letters Vol. 28 (11), pp. 902-904; Le Harzic R. et al. 2009 Applied Optics 48, 3396-400*) .*

Therefore, analysis of the prior art leads to the observation that there is a need for fluorescent PDT compounds exhibiting a high stringency for avoiding sustained skin photosensitivity while being sensitive enough to keep non invasive safety excitation conditions.

Moreover, there is a need for new PDT compounds which are compatible not only with a one-photon excitation but also with two-photons excitation to provide a highly and accurate treatment.

A good fluorescence signal could be another interesting property, in particular if the fluorescence emission itself or the subcellular localization of emission could be indicative of the time-course of the photo-induced apoptosis phenomenon.

The Applicant found that specific cationic triphenylamines derivatives are capable of meeting these needs.

### DESCRIPTION OF THE INVENTION

### Cationic triphenylamine derivatives compounds

The present invention relates to cationic triphenylamines derivatives.
According to the invention, cationic triphenylamines derivatives (or TPA) mean compounds having a common structure presenting the following general formula (A). 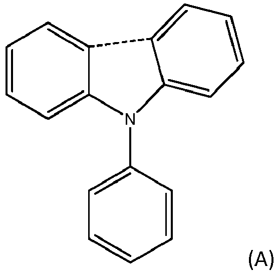 where
---- is an optional covalent bond

The object of the present invention is a cationic triphenylamine derivative presenting the following general formula (I): 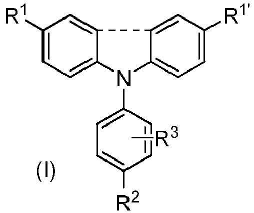 where
---- is an optional covalent bond;
each of R¹, R^{1'}, R² is the same or different and represents 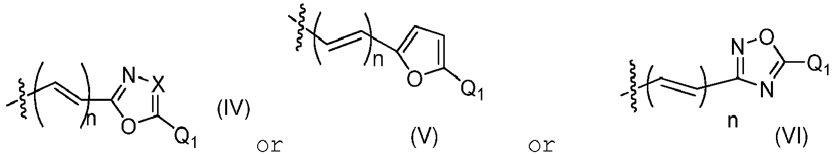 with X= CH or N, n=0 or 1 and Q₁ being :
- an heterocyclic group of formula (i) 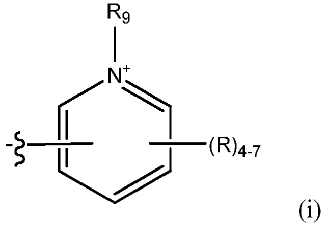 where R₉ represents an hydrocarbon group;
   R₄ to R₇ represent independently an hydrogen or an hydrocarbon group;
- an heterocyclic group of formula (ii), (iii), (iv) or (v) 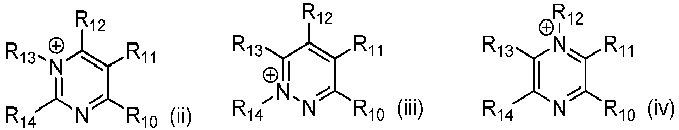 where R₁₀ represents a covalent bond linking said heterocyclic group of formula (ii), (iii) or (iv); R₁₁-R₁₄ represent independently of each other an hydrogen or an hydrocarbon group; 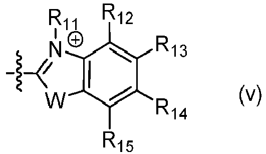 where R₁₁-R₁₅ each represents independently of each other an hydrogen or an hydrocarbon group; W represents an oxygen or NR₁₆ with R₁₆ being an hydrocarbon group;
   R² can also be an hydrogen or a linking group which is a functional group capable of allowing the grafting, by a chemical reaction, of the compound of formula (I) on a biomolecule, or a hydrocarbon group comprising such a functional group;
   R³ represents H or a linking group which is a functional group capable of allowing the grafting, by a chemical reaction, of the compound of formula (I) on a biomolecule, or a hydrocarbon group comprising such a functional group; with the proviso that compound (I) does not represent

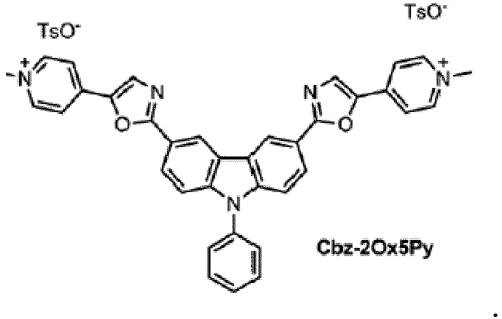

The term "*hydrocarbon group*" means a chain of 1 to 25 carbon atoms, typically 1 to 12 carbon atoms, more typically 1 to 10 carbon atoms, and most typically 1 to 8 carbon atoms. Hydrocarbon groups may have a linear or branched chain structure. Typical hydrocarbon groups have one or two branches, typically one branch. Typically, hydrocarbon groups are saturated. Unsaturated hydrocarbon groups may have one or more double bonds, one or more triple bonds, or combinations thereof. Typical unsaturated hydrocarbon groups have one or two double bonds or one triple bond; more typically unsaturated hydrocarbon groups have one double bond, hydrocarbon groups may be substituted with one or more substituents, which are identical or different, and comprise one or more heteroatoms.

For example, hydrocarbon groups may be alkyl group.

The term "*alkyl group*" means linear or branched carbon radicals in C₁-C₂₅, preferably in C₁-C₁₂, more preferably in C₁-C₈ or in C₁-C₆, even more preferably in C₁-C₄. Typically, they are chosen in the group consisting of methyl, ethyl, n-propyl, isopropyl, n-butyl, isobutyl, sec-butyl and tert-butyl.

Cationic triphenylamine derivatives according to the invention penetrate by themselves in targeted cells. However, it would be interesting to vectorize compound (I) so as to target preferentially specific cancer cells avoiding normal cells.

Therefore, according to an embodiment, R³ and/or R² represents a linking group which is a functional group capable of allowing the grafting, by a chemical reaction, of the compound of formula (I) on a biomolecule, said biomolecule being chosen in the group consisting of folic acid, polyamines such as spermine, spermidine or peptides.

According to an embodiment, the linking group being a functional group is chosen in the group consisting of polyethylene glycol of various lengths, or alkyl chains bearing a terminal functional group such as halogen group or carboxylic acid group, an azido group or a terminal alkyne.

The compound of formula (I) can be grafted on said biomolecule by a chemical reaction such as amidation, Huisgen cycloaddition.

According to a preferred embodiment, cationic triphenylamine derivative does not contain the optional bond.

Of course, the cationic triphenylamine derivative of formula (I) is associated with a counter-ion chosen from the group consisting of tosylate, mesylate, hydrochloride, hydrobromide, tartrate, hydroiodide, fumarate, citrate, trifluoroacetate, ascorbate, triflate, formate, acetate and maleate to ensure ionic neutrality.

The counter-ion and/or the number of the counter ion will be chosen according to the valence so as to maintain the ionic neutrality.

The counter-ion, even if present, is not always represented in the present specification.

According to an embodiment of the present invention, at least two of R¹, R^{1'} or R² are identical.

According to another embodiment of the present invention, at least two of R¹, R^{1'} or R² represent (IV) with Q₁ being (i) or (v).

According to another embodiment when two of R¹, R^{1'} or R² represent (IV) with Q₁ being (i) or (v), W is NR₁₆ and R₁₂-R₁₅ are identical and represent H.

According to another embodiment, the cationic triphenylamine derivatives are compounds TP-2(Ox5Q1) and TP-3(Ox5Q1). 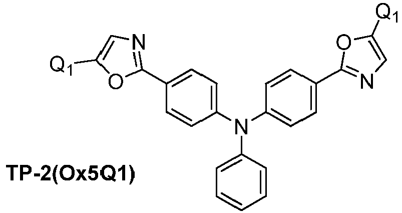 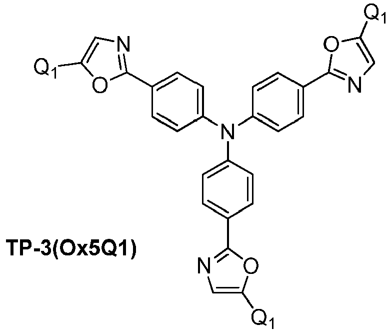 with

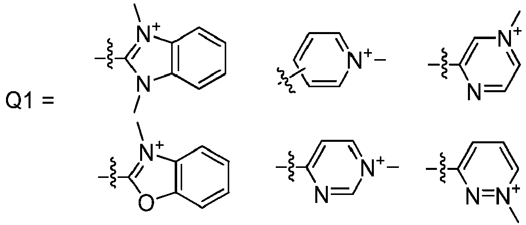

According to a preferred embodiment, the cationic triphenylamine derivatives according to the invention are compounds selected from the group consisting of those of formula (a), (b), (c) or (d) named respectively later on TP30x5Py, TP20x5Py, TP30x5Bzim and TP20x5Bzim.

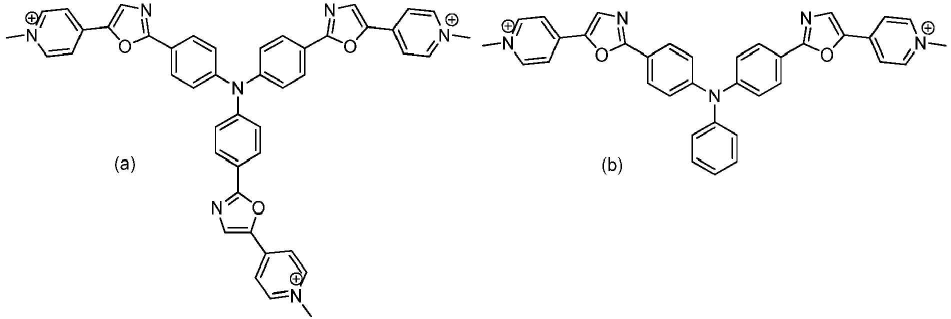

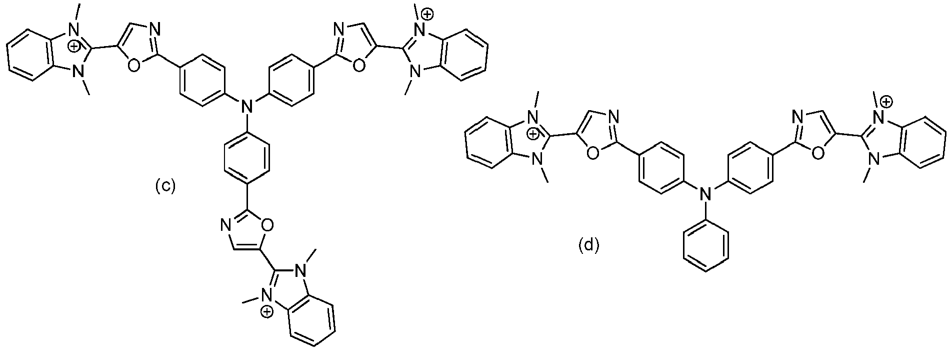

The cationic triphenylamines derivatives described above are highly fluorescent, especially when bound to a target such as nucleic acids, specifically DNA, or a cell organelle. The binding process strongly limits the internal dynamics of TPA, leading to a substantial increase in the fluorescence quantum yield.

Therefore, compared to porphyrins and chlorins, they typically display a higher quantum yield. Indeed, for instance, chlorine e6 is characterized by a quantum yield of 0.008-0.01 in water up to 10 µM.

The cationic triphenylamines derivatives according to formula (I) exhibit a quantum yield in presence of DNA ranging from 0.01 to 0.8, preferably from 0.08 to 0.6. The values of quantum yield were measured using a reference Rhodamine 101 in ethanol according to: Lakowics JR (2006) Principles of Fluorescence Spectroscopy, 3rd Ed., Springer-Verlag New York Inc., New York.

According to the invention, the cationic triphenylamine derivatives described above exhibit a fluence ranging from 10 to 300, preferably from 15 to 200 and more preferably from 22 to 100J/cm² for one photon excitation and a fluence ranging from 50 to 500, preferably from 100 to 400 and more preferably from 150 to 300J/cm² for two photons excitation.

The fluence can be calculated by multiplying the irradiance by the time of light exposure.

The total intensity (or irradiance in mW/cm²) can be measured by using a power meter (Nova II / PD300-3W photodiode sensor or VEGA / 3A thermal head or equivalent).

According to the invention, the cationic triphenylamine derivatives described above present a two-photon cross section which is comprised between 100 and 2000GM, and more preferably between 400 and 1000GM. The cross section can be determined by different methods such as Z-scan, Non Linear Transmission (NLT) or two-photon-induced fluorescence (TPIF) measurements.

### Synthesis of triphenylamine derivatives of formulae TP-2(Ox5Q1) and TP-3(Ox5Q1)

Another object of the present invention is a synthesis of compounds of the present invention, specifically compounds TP-2(Ox5Q1) and TP-3(Ox5Q1) presenting respectively the general formula below. 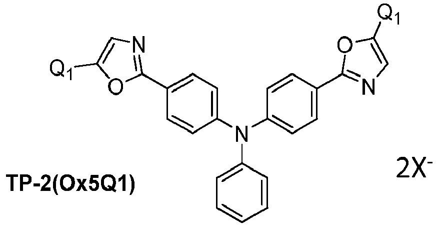 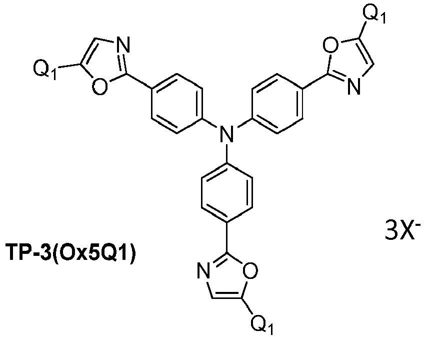 with 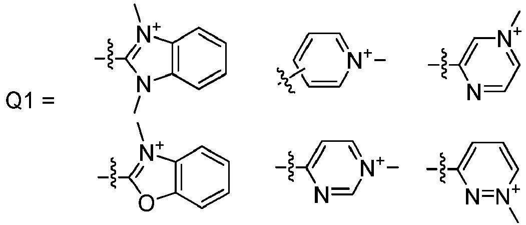 and X⁻ being an anion, for exemple, tosylate.

The compounds of general formula TP-2(Ox5Q1) or TP-3(Ox5Q1) can be prepared according to scheme 1 below.

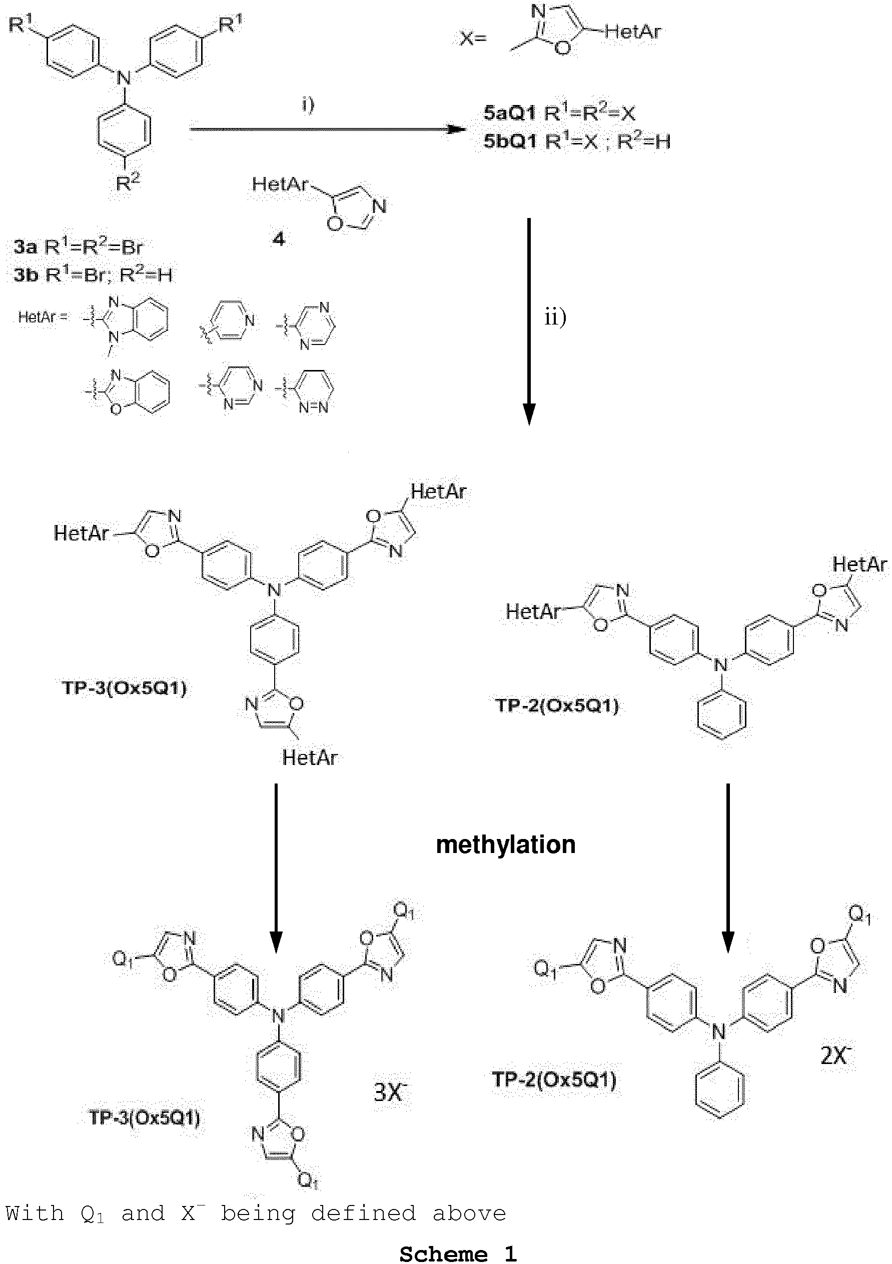

A compound of formula 3a (R¹ and R² are selected in the group consisting of Br, Cl and I) or 3b (R¹ is selected in the group consisting of Br, Cl and I) is placed in a dry protic solvent S1. A compound of formula 4 wherein HetAr is as defined in scheme 1 is then added in a molar ratio ranging from 1 to 5, preferably from 1 to 1.5 with respect to the compound of formula 3a or 3b in presence of a base in a molar ratio ranging from 1 to 6, preferably from 2 to 4 in the presence of a catalyst in an amount ranging from 1 mol% to 15 mol%, preferably from 2mol% and 10mol% relative to the total amount of compound of formula 3a or 3b. The reaction mixture is then heated at a temperature ranging from 70°C to 150°C, preferably from 80 to 130°C, and more preferably at 120°C in a Schlenk flask for example and stirred for a time ranging from 1 to 30 hours, preferably from 2 to 20 hours, and more preferably during 18 hours under inert gas such as argon. The reaction mixture can be concentrated under reduced pressure and the residue can be diluted with an organic solvent. The organic phase can be washed with water, decanted and dried over magnesium sulphate. The compounds are purified (by flash chromatography on silica gel for example). Finally, solid can be dried under vacuum overnight to give a compound of formula 5.

A compound of formula 5a or 5b and a compound chosen in particular among of methyl tosylate or methyl mesylate (or another suitable compound that the man skilled in the art will be able to choose according to the desired salt) in a ratio ranging from 1 to 10, preferably from 1 to 6 equivalents are placed in an aprotic solvent S2. The reaction mixture can then be heated at a temperature ranging from 30 and 150°C, preferably from 40 to 120°C, more preferably at 110°C and stirred for a time ranging from 8 to 30 hours, preferably from 15 to 25 hours, and more preferably during 20 hours. After cooling down to room temperature, the precipitate is filtered, washed with for example hot toluene and diethyl ether to afford the desired salt TP-2(Ox5Q1) or TP-3(Ox5Q1) depending on the starting material 5a or 5b.

The starting compounds of formula 3a, 3b and 4 are commercially available or can be prepared according to methods known to the person skilled in the art.

According to the invention, the dry protic solvent S1 is for example 1,4-dioxane.

According to the invention, the aprotic solvent S2 is chosen in the group consisting of apolar solvents such as alkanes such as cyclohexane, pentane or aromatic solvents such as xylene, toluene and preferably is toluene.

According to the invention, the base is chosen in the group consisting of inorganic bases such as potassium carbonate, cesium carbonate, sodium tert-butoxide or lithium tert-butoxide and preferably is lithium tert-butoxide.

According to the invention, the catalyst is chosen in the group consisting of palladium(0) species such as Pd(OAc)2 and a phosphine such as triarylphosphine, trialkylphosphine or tetrakis(triphenylphosphine)palladium(0), and preferably is tetrakis(triphenylphosphine)palladium(0).

The residue can be diluted with an organic solvent such as ethyl acetate and the organic phase can be washed with water.

The compounds can be purified by flash chromatography on silica gel or by recrystallization.

### Solution and pharmaceutical composition

Another object of the present invention is a solution comprising at least a cationic triphenylamine derivative according to formula (I) and at least a solvent.

Another object of the present invention is a pharmaceutical composition comprising a cationic triphenylamine derivative according to the invention and a pharmaceutically acceptable carrier.

A "solution", as used herein, is defined as comprising at least a cationic triphenylamine derivative according to the invention and at least a solvent and comprises necessarily a counter-ion to ensure ionic neutrality.

A "pharmaceutical composition", as used herein, is defined as comprising at least a cationic triphenylamine derivative according to the invention, and at least one pharmaceutically acceptable carrier and comprising necessarily a counter-ion to ensure ionic neutrality.

As used herein, the term "*pharmaceutically acceptable carrier*" refers to a carrier medium which does not interfere with the effectiveness of the biological activity of the active ingredients and which is not excessively toxic to the hosts at the concentrations at which it is administered. The carrier(s) may be particulate, so that the compositions are, for example, in tablet or powder form. The carrier(s) may be liquid, with the compositions being, for example, an oral syrup or injectable liquid. In addition, the carrier(s) may be gaseous, so as to provide an aerosol composition useful in, *e.g*., inhalatory administration.

The term "*effective amount*" refers to any amount of pharmaceutical composition, which is sufficient to fulfill its intended purpose(s) (*e.g*., the purpose may be the apoptosis in cancer cells in a biological system or in a subject).

As used herein, the term *"subject"* refers to a human or another mammal (*e.g*., mouse, rat, rabbit, hamster, dog, cat, cattle, swine, sheep, horse or primate). In many embodiments, the subject is a human being. In such embodiments, the subject is often referred to as an *"individual",* or a *"patient"* if the subject is afflicted with a disease or clinical condition. The terms "subject", "individual" and "patient" do not denote a particular age, and thus encompass adults, children and newborns.

According to an embodiment, the solution comprises a cationic triphenylamine derivative selected from the group consisting of TP-2(Ox5Q1), TP-3(Ox5Q1), preferably TP30x5Py, TP20x5Py, TP30x5Bzim, TP20x5Bzim and mixture(s) thereof, and a solvent.

The solvent will be chosen so as to solubilize the cationic triphenylamine derivative of the invention.

The man skilled in the art will be able to choose the solvent suitable to the use of the solution.

For example, DMSO with a maximum final concentration 1% (v/v) to maintain cell viability can be used.

Another object of the present invention is a pharmaceutical composition comprising a cationic triphenylamine derivative selected from the group consisting of compounds of formula (21) defined below, compounds of formula (I) defined above and mixture(s) thereof and a pharmaceutically acceptable carrier. 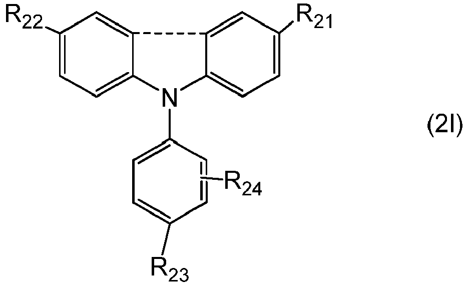 in which:
----- represents an optional covalent bond;
R₂₄ represents a hydrogen atom or a linking group, in which case:
1) if R₂₄ represents a hydrogen atom, then:
   R₂₁ represents a linking group or a group of formula (II)' below: 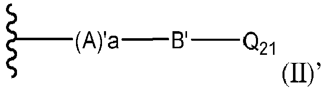 in which:
   Q₂₁ represents:
   a heterocyclic group of formula (i)' below: 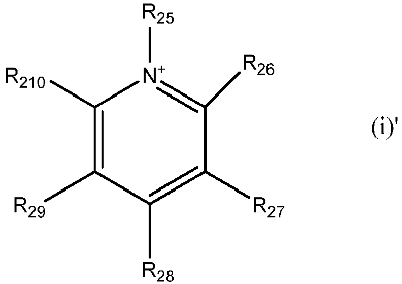
   in which R₂₅ represents a hydrocarbon group; any one of R₂₆-R₂₉ and R₂₁₀ represents a covalent bond linking the said heterocyclic group to B, while the others from R₂₆-R₂₉ and R₂₁₀ represents, independently of each other, a hydrogen atom or a hydrocarbon group; or
   a heterocyclic group of formula (ii)' or (iii)' below: 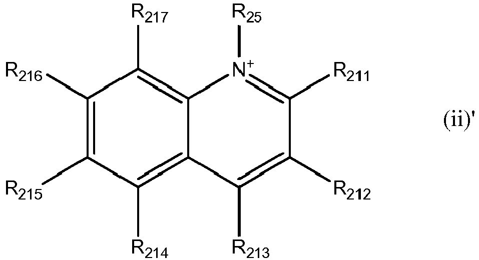 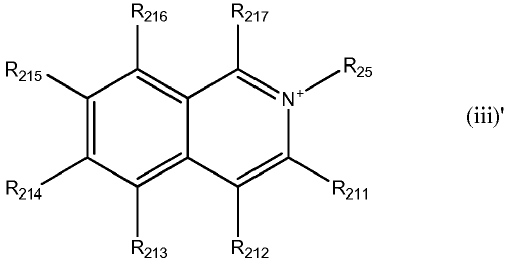
   in which R₂₅ represents a hydrocarbon group; any one of R₂₁₁ to R₂₁₇ represents a covalent bond linking the said heterocyclic group to B, while the others from R₂₁₁ to R₂₁₇ represent, independently of each other, a hydrogen atom or a hydrocarbon group, it being possible for R₂₁₂, R₂₁₄ and R₂₁₆ to also form, respectively with R₂₁₁ and/or R₂₁₃, R₂₁₃ and/or R₂₁₅, and with R₂₁₅ and/or R₂₁₇, a bridging group; or
   a heterocyclic group of formula (iv)' below: 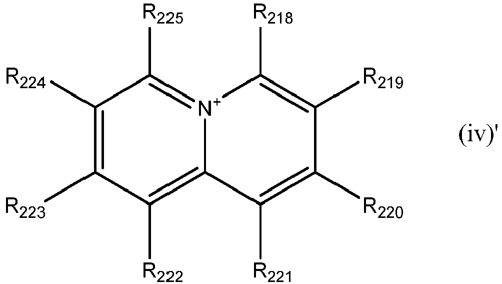
   in which any one of R₂₁₈ to R₂₂₅ represents a covalent bond linking the said heterocyclic group to B, while the others from R₂₁₈ to R₂₂₅ represent, independently of each other, a hydrogen atom or a hydrocarbon group, it being possible for R₂₁₉, R₂₂₁, R₂₂₃ and R₂₂₅ to also form, respectively with R₂₁₈ and/or R₂₂₀, R₂₂₀ and/or R₂₂₂, R₂₂₂ and/or R₂₂₄, and with R₂₂₄ and/or R₂₁₈, a bridging group; or
   a heterocyclic group of formula (v)' below: 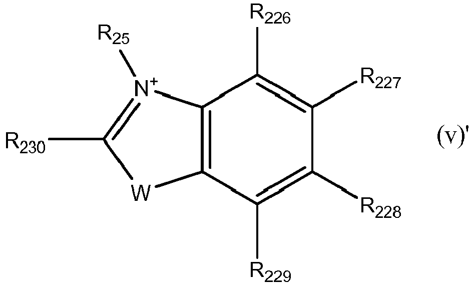
   in which R₂₂₅ represents a hydrocarbon group; W represents an oxygen or sulphur atom or a group -N(R₂₃₁)- in which R₂₃₁ is a hydrogen atom or a hydrocarbon group, or a group - C(R₂₃₁)(R₂₃₂)- in which R₂₃₁ and R₂₃₂ are, independently of each other, a hydrogen atom or a hydrocarbon group; R₂₃₀ represents a covalent bond linking the said heterocyclic group to B, while R₂₂₆ to R₂₂₉ represent, independently of each other, a hydrogen atom or a hydrocarbon group, it being possible for R₂₂₇ to also form with R₂₂₆ and/or R₂₂₈ a bridging group, it being possible for R₂₂₈ itself to form with R₂₂₉ a bridging group;
   a is equal to 0 (in which case A' is absent) or 1 (in which case A' is present);
   A' and B' represent the groups below: 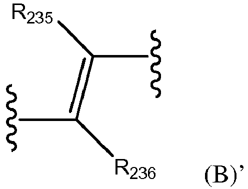 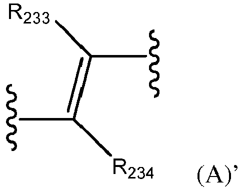
   in which R₂₃₃ to R₂₃₆ represent, independently of each other, a hydrogen atom or a hydrocarbon group, it being possible for each of R₂₃₃ and R₂₃₆ to form with R₂₃₄ and/or R₂₃₅ a bridging group;
   when R₂₁ represents a group of formula (II)' above, then R₂₂ also represents a group of formula (II)' above while, when R₂₁ represents a linking group, then R₂₂ represents a group of formula (III)' below: 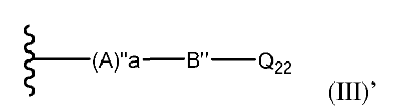
   in which:
   Q₂₂ represents:
      a heterocyclic group corresponding to any one of the formulae (i)' to (v)' above; or
      a heterocyclic group of formula (vi)' below: 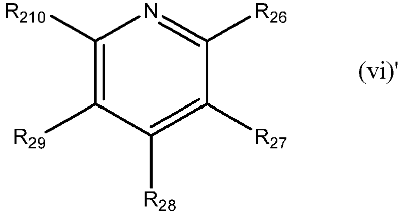
   in which R₂₆-R₂₉ and R₂₁₀ have the same meaning as in the formula (i)' above; or
   a heterocyclic group of formula (vii)' or (viii)' below: 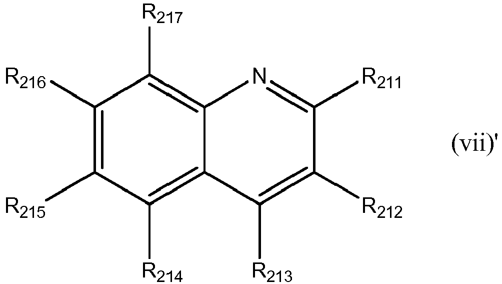 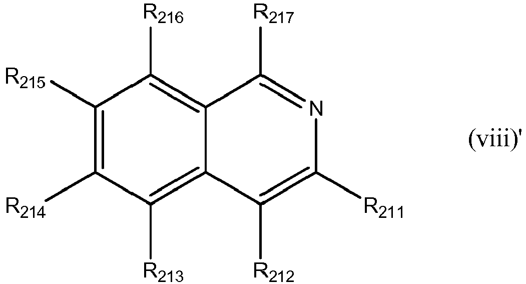
   in which R₂₁₁ to R₂₁₇ have the same meaning as in the formulae (ii)' and (iii)' above; or
   a heterocyclic group of formula (ix)' below: 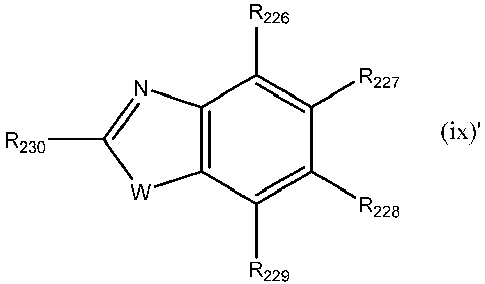
   in which W, R₂₂₆ to R₂₃₀ have the same meaning as in the formula (v)' above;
   a, A" and B" have the same meaning as above;
   when R₂₁ represents a linking group, then R₂₃ represents a group of formula (III)' above while, when R₂₁ represents a group of formula (II)' above, then R₂₃ represents a hydrogen or halogen atom, a hydrocarbon group or a group of formula (II)' above;
2) if R₂₄ represents a linking group, then R₂₁ and R₂₂ represent a group of formula (III)' above while R₂₃ represents a hydrogen or halogen atom, a hydrocarbon group or a group of formula (III)' above;
   in which the linking group is a functional group capable of allowing the grafting, by a chemical reaction, of the compound on a biomolecule, or a hydrocarbon group comprising such a functional group, and in which each of the abovementioned hydrocarbon groups may be substituted with one or more substituents, which are identical or different, and comprise one or more heteroatoms.

According to a preferred embodiment, the pharmaceutical composition comprises a cationic triphenylamine derivative of formula (2IA) corresponding to the formula (2I) but in which Q₂₁ cannot be an heterocycle of formula (ii)', (iii)', (iv)' and in which it is not possible for each of R₂₃₃ and R₂₃₆ to form with R₂₃₄ and/or R₂₃₅ a bridging group.

According to preferred embodiment, the pharmaceutical composition comprises at least a compound chosen in the group consisting of TP-2(Ox5Q1), TP-3(Ox5Q1), preferably TP30x5Py, TP20x5Py, TP30x5Bzim, TP20x5Bzim, or a compound chosen in the group consisting of TP3Pyo, TP2Pyo, Cbz2Py, Cbz3Py, TP2BZim, TP3BZim, TP2Py, TP3Py and mixture thereof and a pharmaceutical acceptable carrier.

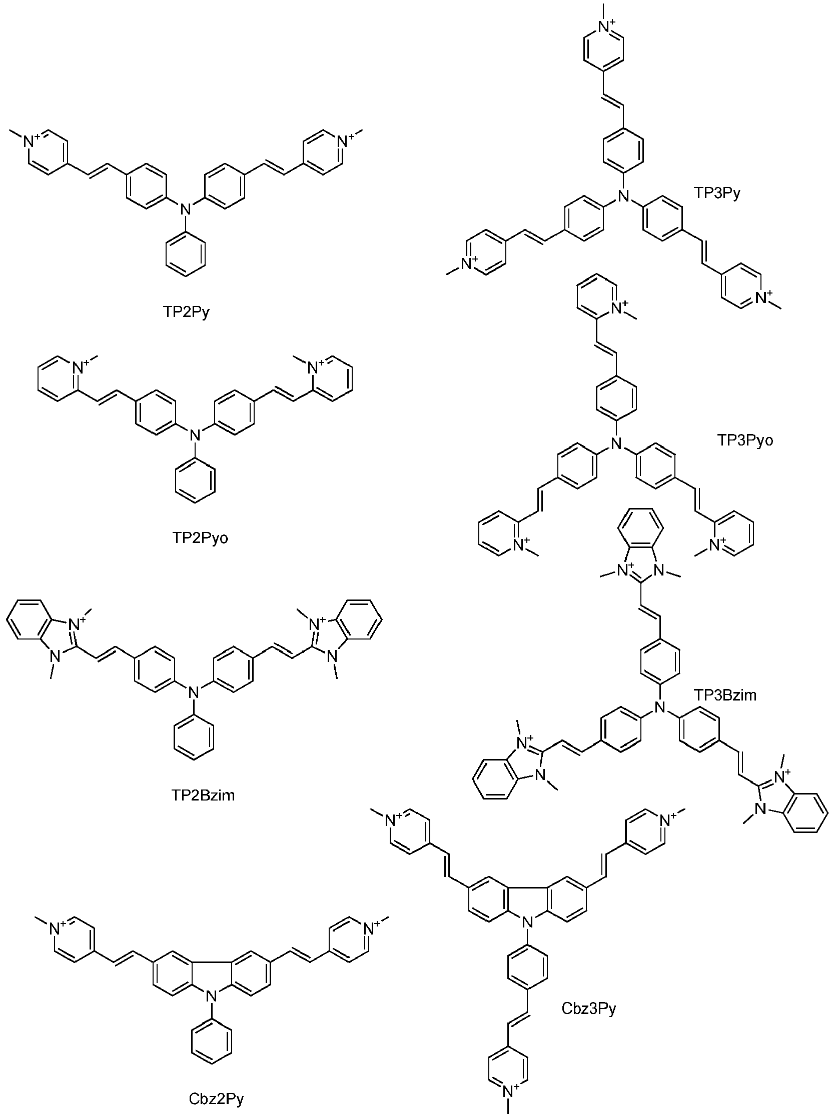

The pharmaceutical composition may be in the form of a liquid (solutions, suspensions or other like form) composition or a solid composition.

According to its form (liquid or solid), the pharmaceutical composition may be intended for different administration routes such as topical administration, parenteral administration or oral administration.

According to a preferred embodiment, when the pharmaceutical composition is in liquid form, it is intended for parenteral administration. The parenteral preparation can be enclosed in ampoules, disposable syringes or multiple dose vials made of glass or plastic.

According to the invention, the pharmaceutical composition contains an effective amount of the compound (I), (21) or (2IA) such that a suitable dosage will be obtained.

### Use

Another object of the invention is the use of cationic triphenylamine derivative of formula (I), (21) or (2IA) or mixture thereof or of a pharmaceutical composition comprising cationic triphenylamine derivative of formula (I), (21) or (2IA) and a pharmaceutically acceptable carrier as photo active agent in dynamic phototherapy.

According to the invention, "dynamic phototherapy" means a method involving the introduction of an exogen molecule as a PDT compound (i.e cationic triphenylamines derivatives of the invention) in a subject or a biological sample.

According to the invention, the PDT compound induces cell death under light illumination (via a one or two-photons absorption process) preferably by generating ROS. This ROS generation can result from a direct mechanism (i.e generation of singlet oxygen by energy transfer from the triplet state of the photosensitizer or photodegradation of the PDT compounds in organism) or the ROS generation can also result from an indirect mechanism (i.e loss of membrane potential of mitochondria).

According to a preferred embodiment, an object of the invention is the use of a cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and mixture thereof as photo active agent in dynamic phototherapy.

Another object of the present invention is the use of a pharmaceutical composition comprising cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and a pharmaceutical acceptable carrier as photo active agent in dynamic phototherapy.

According to the invention, cationic triphenylamine derivative of formula (I), (2I) or (2IA) or a pharmaceutical composition comprising said cationic triphenylamine derivative can be used in one-photon dynamic phototherapy, into the 400-700 range, preferably into the 400-600 nm range for excitation or in two-photons dynamic phototherapy into the 700-930 nm, preferably into the 840-900 nm range for the excitation.

Indeed, experimental data have shown that the cationic triphenylamine derivative of formula (I), (21) or (2IA) according to the invention can be used not only as photo active agent in one-photon dynamic phototherapy but also in two-photons dynamic phototherapy.

According to a preferred embodiment, cationic triphenylamine derivative of formula (I),(2I) or (2IA) or composition comprising said cationic triphenylamine derivative are used in two-photons dynamic phototherapy since the excitation wavelength in the near-infra red region of the spectrum offers deeper penetration in the tissues, and therefore, offers a better treatment.

When the cationic triphenylamine derivatives are used as a photoactive agent in two-photons dynamic phototherapy, the wavelength is ranged from 840 nm to 900 nm.

Another object of the present invention is the use of cationic triphenylamine derivative of formula (I),(2I) or (2IA) as photo-dependent inducers of apoptosis on living cells.

According to a preferred embodiment, an object of the invention is the use of a cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and mixture thereof as photo-dependent inducer of apoptosis on living cells.

Another object of the present invention is the use of a pharmaceutical composition comprising cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and a pharmaceutical acceptable carrier as photo-dependent inducer of apoptosis on living cells.

Indeed, it has been underlined that rapid cell death (on the minute scale) can be revealed after selective excitation of triphenylamine derivatives according to the invention using one or two-photon excitation process. The photo-induced cell death is concomitant to ROS (reactive Oxygen Species) generation as revealed by the H2DCF-DA probe and actually corresponds to an apoptosis mechanism as revealed by the PARP cleavage and the externalization of phosphatidyl serine, two well-known hallmarks of apoptosis.

Moreover, due to their fluorescent properties, the cationic triphenylamine derivatives according to the invention allow a simultaneous monitoring of the overall apoptosis process on the same sample by following the re-localization of the fluorescent signal (from the cytoplasm to the nucleus) or/and by directly monitoring the formation of membrane blebs (another hallmark of apoptosis).

Therefore, another object of the present invention is the use of cationic triphenylamine derivative formula (I), (2I) or (2IA) as fluorescent apoptotic tracers when these derivatives - exposed to light - trigger by themselves the cell apoptosis.

Another object of the present invention is the use of cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and mixture thereof as fluorescent apoptotic tracers.

Another object of the present invention is the use of a pharmaceutical composition comprising a cationic triphenylamine derivative selected from the group consisting of compounds TP-2(Ox5Q1), TP-3(Ox5Q1), preferably TP30x5Py, TP20x5Py, TP30x5Bzim, TP20x5Bzim, or a cationic triphenylamine derivative selected from the group consisting of compounds TP3Pyo, TP2Pyo, Cbz2Py, Cbz3Py, TP2BZim, TP3BZim, TP2Py, TP3Py and a pharmaceutical acceptable carrier as fluorescent apoptotic tracers.

Still another object of the present invention is the use of cationic triphenylamine derivative formula (I), (21) or (2IA) or a pharmaceutical composition comprising said compounds as photodependant inducer of apoptosis and fluorescent apoptotic tracers of the induced apoptosis.

When the cationic triphenylamine derivative of formula (I), (2I) or (2IA) is used as photo-dependent inducers of apoptosis on living cells, the molar concentration of said triphenylamine derivative is ranged from 10nM to 10µM, preferably from 20nM to 2µM in the solution or pharmaceutical composition of the present invention.

Indeed, it has been shown that such concentrations are below the dark toxicity values defining the intrinsic toxicity of the compound in the absence of any illumination by light. Morevover, the cationic triphenylamine derivatives of formula (I), (21) or (2IA) have the benefit of being non photosensitive to natural light.

However, when the cationic triphenylamine derivative of formula (I), (21) or (2IA) is used as fluorescent apoptotic tracers for cell studies (i.e. as visualized by the re-localization of the fluorescence signal from cytoplasm to the nucleus as mentioned above), the molar concentration of said triphenylamine derivative must be above 50 nM and preferably above 200nM.

Therefore, according to a preferred embodiment, the molar concentration of said triphenylamine derivative is ranged from 200nM to 2µM so as to follow apoptosis phenomenon by fluorescence.

Another object of the invention is a cationic triphenylamine derivative of formula (I), (21) or (2IA) or a pharmaceutical composition comprising said cationic triphenylamine derivative for use as a drug.

Another object of the invention is a cationic triphenylamine derivative of formula (I), (21) or (2IA) or a pharmaceutical composition comprising said cationic triphenylamine derivative for use in the treatment of cancer, preferably by dynamic phototherapy.

Another object of the invention is the use of a cationic triphenylamine derivative of formula (I), (21) or (2IA) or a pharmaceutical composition comprising said cationic triphenylamine derivative in the manufacture of a medicament for the treatment of cancer, preferably by dynamic phototherapy.

According to a preferred embodiment, the cancers to be treated are chosen in the group consisting of cervical cancer, breast cancer, colorectal cancer, skin cancer especially non-melanoma skin cancer; head cancer, neck cancer, esophagus cancer, pancreas cancer, bladder cancer or prostate cancer.

Another object of the present invention is the use of cationic triphenylamine derivative of formula (I), (21) or (2IA) or a pharmaceutical composition comprising said cationic triphenylamine derivative as a cleaning agent for postoperative wounds, for example using spray and subsequent light illumination. In the case if (i) TPAs specifically target tumor cells in patients by their own or if (ii) TPAs specifically target tumor cells by a process based on vector coupling, there is possibility to observe the area concerned by fluorescence so as to delineate the targeted tissues to be light-treated or removed.

Another object of the invention is the cationic triphenylamine derivative of formula (I), (21) or (2IA) or a pharmaceutical composition comprising said cationic triphenylamine derivative for use in the treatment of psoriaris, and superficial mycoses such as fungi or pityriasis.

Another object of the invention is the use of a cationic triphenylamine derivative of formula (I), (21) or (2IA) or a pharmaceutical composition comprising said cationic triphenylamine derivative in the manufacture of a medicament for the treatment of psoriaris, and superficial mycoses such as fungi or pityriasis.

Another object of the present invention is a method for treating cancer by means of PDT comprising the following steps:
- administrating to a patient an effective amount of cationic triphenylamine derivative of formula (I), (21) or (2IA) or a pharmaceutical composition comprising said cationic triphenylamine derivative;
- illuminating the area to be treated by means of photon(s) excitation thereby inducing apoptosis.

Another object of the present invention is a method for treating cancer by means of PDT comprising the following steps:
- administrating to a patient an effective amount of cationic triphenylamine derivative of formula (I), (21) or (2IA) or a pharmaceutical composition comprising said cationic triphenylamine derivative;
- illuminating the area to be treated by means of photon(s) excitation thereby inducing apoptosis; and
- following the apoptosis phenomenon by fluorescence.

The step of following the apoptosis phenomenon by fluorescence enables to stop the illumination when the apoptosis is completed.

According to an embodiment, the effective amount of compounds (I), (2I) or (2IA) or of a pharmaceutical composition comprising said compounds is administrated by parenteral route.

According to another embodiment, the area to be treated is illuminated by means of one photon excitation or a two-photons excitation, preferably by means of two-photons excitation.

### DESCRIPTION OF DRAWINGS

Embodiments of the invention are described by way of example with reference to the accompanying drawings, in which:
Figures 1 show epifluorescence (A) and confocal (B) images of fixed HeLa cells treated with TPA (left: TP2Py & right: TP3Bzim). The corresponding transmission images are shown below the fluorescence images.
Figures 2 represent epifluorescence imaging of living HeLa cells treated with TPA, TP2Py (A) or TP3Bzim (B) (under continuous illumination). The corresponding transmission images are shown below the fluorescence images.
Figures 3 represent the co-localization of TP2Py with the mitotracker Green FM by using confocal microscopy images of living MCF-7 cells treated with both TP2Py and the MitoTracker Green FM.
Figures 4 show epifluorescence images of living HeLa cells treated with TP2Py showing that the duration of the preincubation does not influence the phenomenon ((left panel) initial localization after 16-hours preincubation; (right panel) same sample as in (left panel) but 15 min after illumination).
Figures 5 represent time-dependent confocal fluorescence imaging of living MCF7 cells treated with (A) TP2Py or (B) TP-3Bzim, (C) quantification of the fluorescence emission intensity of TP2Py as a function of time; (D) fluorescence emission spectra of TP2Py in distinct ROIs (ROI = Region Of Interest), (E) confocal image showing the selected ROIs used in panels C and D.
Figure 6 is confocal fluorescence imaging of living MCF-7 cells treated with a neutral TPA: TP3^{N}Bzim used as a control.
Figures 7 represent (A;B) confocal images of living MCF-7 cells treated with TP2Py at (A) high irradiance or (B) low irradiance; (C) Fluorescence emission intensity in ROIs corresponding to the nucleus as a function of time.
Figures 8 show confocal images of living MCF-7 cells treated with different concentration of TP2Py (A) 2µM (B) 200nM and (C) 20nM; Fluorescence emission intensity in ROIs corresponding to the nucleus as a function of time (A1) C=2µM and (B1) C=200nM.
Figures 9 represent cell viability (in % of control treated in the absence of TPA) as a function of time illumination (for a given TPA concentration) ((A) for MCF7 cells and (B) for HeLa cells) or as a function of TPA concentration (for a given time illumination) ((C) for MCF7 cells and (D) for HeLa cells).
Figures 10 show (A) two photon fluorescence images as a function of time of living MCF-7 cells treated with TP2Py; (B) Transmission image corresponding to t = 30 min.
Figures 11 represent (A) Western blot analysis of HeLa cells treated with 2 µM TP2Py or not (DMSO only); (B) Transmission (top) and epifluorescence (bottom) images of the cells after incubation with the TP2Py compound without any further light illumination treatment (left) or with a light illumination treatment of 15 min (middle) or 90 min (right).
Figures 12 and 13: Flow cytometer analysis of MCF-7 cells treated under various conditions, showing that TPA induces both the loss of plasma membrane polarity (Fig. 12: Annexin V staining) and the production of ROS (Reactive Oxygen Species) (Fig. 13: H₂DCF-DA test).

### EXAMPLES

### Example 1: Synthesis of triphenylamine derivatives TP-3(OxSPy) and TP-2(Ox5Py) and TP-3(Ox5BZim)

### Synthesis of TP-3(Ox5Py) and TP-2(Ox5Py)

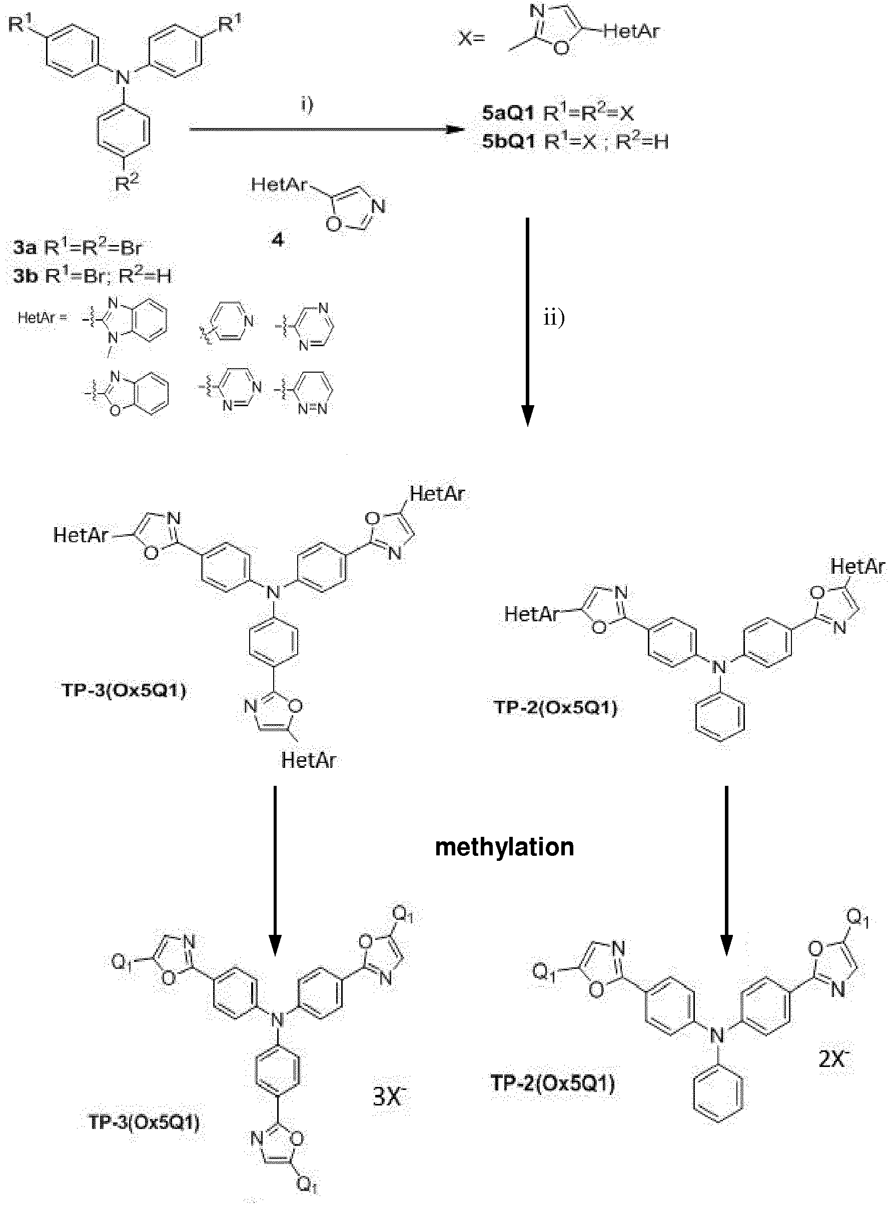

with

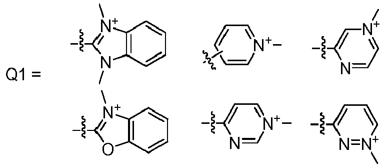

### Preparation of compound 5aPy

A mixture of tris-bromotriphenylamine **3a** (123 mg, 0.25 mmol, 1 equiv.), 5-pyridyloxazole (112 mg, 0.77 mmol, 3 equiv.), lithium *tert*-butoxide (92 mg, 1.1 mmol, 4.5 equiv.), tetrakis(triphenylphosphine)palladium(0) (44 mg, 0.04 mmol, 0.15 equiv.) in dry dioxane (2 mL) was stirred for 2 h at 120°C in a sealed tube. Dichloromethane and water were added. After decantation, the resulting organic layer was washed with brine, dried over MgSO₄, filtered and concentrated to dryness. The crude residue was purified by column chromatography (dichloromethane/methanol = 100/0 to 90/10), dissolved in a minimal amount of dichloromethane and precipitated with diethyl ether to afford the desired compound **5aPy** in 35 % yield (60 mg, 0.09 mmol) as a yellow solid.
¹H NMR (300 MHz, CDCl₃) δ 8.69 (d, *J* = 5. 0, 2H), 8.09 (d, *J* = 8.6, 2H), 7.67 (s, 1H), 7.58 (d, *J* = 5.7, 2H), 7.30 (d, *J* = 8.6, 2H) ¹³C NMR (75 MHz, CDCl₃) δ 162.2, 150.5, 148.8, 148.6, 134.8, 128.2, 127.1, 124.6, 122.3, 117.9
MS (ES+) m/z 678.3
Anal. (C₄₂H₂₇N₇O3.0.5H₂O) Calcd: C, 73.46; H, 4.11; N, 14.28 Found : C, 73.62; H, 3.89; N, 13.96

### Preparation of TP-3(Ox5Py)

A suspension of compound **5aPy** (40 mg, 0.06 mmol, 1 equiv.) and methyl tosylate (50 mg, 0.26 mmol, 4.5 equiv.) in toluene (4 mL) was stirred for 12 h at reflux. After cooling down to room temperature, the reaction mixture was sonicated and put at 4°C for 2 days. The resultant fine solid was washed with hot toluene and diethyl ether to afford the desired salt as an orange solid (52 mg, 0.04 mmol) in 71% yield.
¹H NMR (300 MHz, DMSO-*d⁶*) δ 9.01 (d, *J* = 6.1, 6H), 8.61 (s, 3H), 8.46 (d, *J* = 6.3, 6H), 8.25 (d, *J* = 8.6, 6H), 7.47 (d, *J* = 7.9, 6H), 7.37 (d, *J* = 8.2, 6H), 7.10 (d, *J* = 7.8, 6H), 4.30 (s, 9H), 2.28 (s, 9H) ¹³C NMR (75 MHz, DMSO) δ 163.9, 148.9, 146.1, 146.0, 145.7, 140.4, 137.7, 134.6, 128.9, 128.1, 125.51, 124.7, 121.2, 120.5, 47.2, 20.8
MS (ES+) m/z 241.0 HRMS (ESI) calcd for C₅₂H₄₃N₇O₆S [M+OTs]²⁺ 446.6498, found 446.6481

### Preparation of compound 5bPy

A mixture of bis-bromotriphenylamine **3b** (426 mg, 1 mmol, 1 equiv.), 5-pyridyloxazole (386 mg, 2.6 mmol, 2.5 equiv.), lithium *tert*-butoxide (338 mg, 4.2 mmol, 4 equiv.), tetrakis(triphenylphosphine)palladium(0) (122 mg, 0.1 mmol, 0.10 equiv.) in dry dioxane (10 mL) was stirred for 21 h at 120°C in a sealed tube. Ethyl acetate and water were added. After decantation, the resulting organic layer was washed with brine, dried over MgSO₄, filtered and concentrated to dryness. The crude residue was purified by column chromatography (dichloromethane/methanol = 100/0 to 90/10), dissolved in a minimal amount of dichloromethane and precipitated with a small quantity of diethyl ether and a large quantity of pentane to afford the desired compound **5bPy** in 26 % yield (147 mg, 0.27 mmol) as a bright yellow solid.
¹H NMR (300 MHz, CDCl₃) δ 8.66 (d, *J* = 5. 4, 4H), 8. 00 (d, *J* = 8.7, 4H), 7.63 (s, 2H), 7.54 (d, *J* = 5.4, 4H), 7.41 - 7.30 (m, 2H), 7.25 - 7.13 (m, 7H) ¹³C NMR (75 MHz, CDCl₃) δ 162.6, 150.5, 149.6, 148.4, 146.3, 135.1, 130.0, 128.0, 127.2, 126.3, 125.3, 123.5, 121.2, 118.0
MS (ES+) m/z 534.2

### Preparation of TP-2(Ox5Py)

A suspension of compound **5bPy** (40 mg, 0.07 mmol, 1 equiv.) and methyl tosylate (42 mg, 0.22 mmol, 3 equiv.) in toluene (4 mL) was stirred for 18 h at reflux. After cooling down to room temperature, the orange precipitate was filtered, washed with hot toluene and diethyl ether to afford the desired salt as an orange solid (60 mg, 0.06 mmol) in 89% yield.
¹H NMR (300 MHz, DMSO-*d⁶*) δ 8.99 (d, *J* = 6.2, 4H), 8.58 (s, 2H), 8.43 (d, *J* = 6.2, 4H), 8.17 (d, *J* = 8.1, 4H), 7.47 (d, *J* = 7.5, 4H), 7.35 - 7.15 (m, 9H), 7.10 (d, *J* = 7.3, 4H), 4.29 (s, 6H), 2.28 (s, 6H) ¹³C NMR (75 MHz, DMSO-*d⁶*) δ 164.1, 149.6, 145.9, 145.8, 145.4, 140.4, 137.5, 134.7, 130.3, 128.9, 128.7, 128.2, 128.0, 126.5, 125.5, 123.0, 120.4, 119.8, 47.2, 20.8
MS (ES+) m/z 282.3 HRMS (ESI) calcd for C₃₆H₂₉N₅O₂ [M⁺] 563.2321, found 563.2304

### Synthesis of TP-3(Ox5Bzim)

A mixture of tris-bromotriphenylamine **3a** (123 mg, 0.25 mmol, 1 equiv.), 5-N-methylbenzimidazoyloxazole (600 mg, 3 mmol, 3 equiv.), lithium tert-butoxide (460 mg, 6 mmol, 6 equiv.), tetrakis(triphenylphosphine)palladium(0) (180 mg, 10mol%) in dry dioxane (5 mL) was stirred overnight at 120°C in a sealed tube. Dichloromethane and water were added. After decantation, the resulting organic layer was washed with brine, dried over MgSO₄, filtered and concentrated to dryness. The crude residue was purified by column chromatography (dichloromethane/methanol = 100/0 to 90/10), dissolved in a minimal amount of dichloromethane and precipitated with diethyl ether to afford the desired compound **5aBzim** in 83% yield (700 mg, 0.83 mmol) as a yellow solid.

A suspension of **5aBzim** (144 mg, 0.172 mmol, 1 equiv.) and methyl tosylate (144.19 mg, 0.77 mmol, 4.5 equiv.) in toluene (15 mL) was stirred for 24 h at reflux. After cooling down to room temperature, the yellow precipitate was filtered, washed with hot toluene and diethyl ether to afford the desired salt as a yellow solid (66 mg, 0.072 mmol) in 84% yield.
¹H NMR (300 MHz, DMSO) δ 8.66 (s, 3H), 8.25 (d, *J* = 9, 6H), 8.16 (m, 6H), 7.74 (m, 6H), 7.44 (d, *J* = 9, 6H), 7.38 (d, *J* = 9, 6H), 7.08 (d, *J* = 9, 6H), 4.27 (s, 18H), 2.25 (s, 9H) ¹³C NMR (75 MHz, DMSO) 164.1, 149.1, 145.7, 139.1, 138.0, 137.6, 134.5, 132.1, 129.2, 128.9, 128.1, 127.1, 125.5, 120.8, 113.4, 33.7, 20.8
MS (ES+) m/z 293.9 HRMS (ESI) calcd for C₅₄H₄₅N₁₀O₃ [M⁺] 881.3676, found 881.3661

### Material

The following instrument set-up for fluorescence imaging was used in examples 2-10.

### One photon images

One-photon epifluorescence and confocal images were obtained using a SP2 confocal microscope (Leica MicroSystem) equipped with an incubation chamber (37°C, CO₂ 5%). Excitation and emission filters for epifluorescence were 450-490 nm and 515-700 nm, respectively. A continuous laser line (458 nm) was used for excitation in the confocal mode.

### Two photons images

A similar set up was used for two-photon images except that the excitation source was a 80-MHz mode-locked Mai Tai® Ti:Sapphire tunable laser (720-920 nm, 100 fs laser pulse; Spectra Physics, Mountain View, California) tuned to 860 nm.

For each experiment, the total intensity, called irradiance (in mW/cm²) was measured using a power meter (Nova II / PD300-3W photodiode sensor or VEGA / 3A thermal head).

### Example 2: Differential properties of TP2Py and TP3Bzim compounds in terms of cellular localization between fixed and living cells.

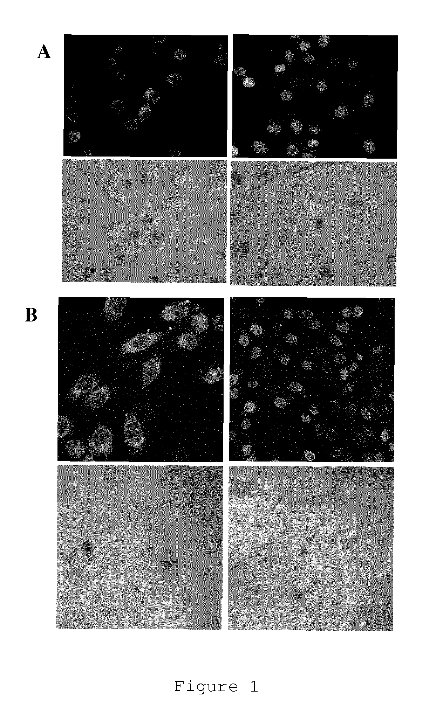

When HeLa cells were treated with TPA (2 µM TP2Py - λ_{max,emission} = 634 nm - or TP3Bzim - λ_{max,emission} = 572 nm - for 2 hours) and fixed using formaldehyde in Fig. 1; (the same result was found using ethanol), epifluorescence and confocal imaging clearly showed a nuclear localization of the TPA compound (Fig. 1), in accordance with the propensity of these compounds to bind in the DNA minor groove *(*Allains et al. 2007 ChemBioChem 8, 1-11). Similar observations were done with MCF7 cells.

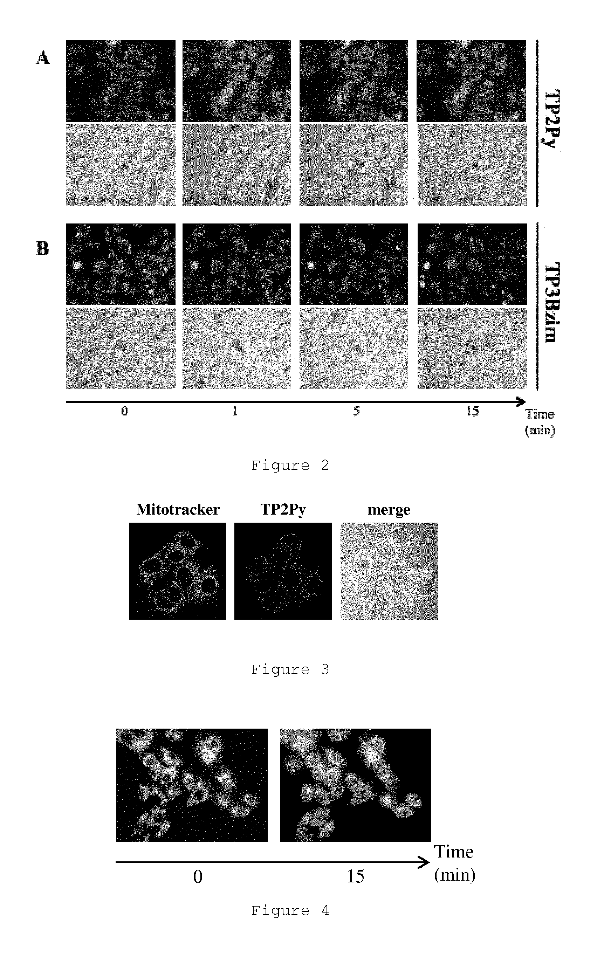

Intriguingly, the labelling of living cells by TPAs led to a completely different pattern of fluorescence distribution in cells. After the 2 hour-incubation step, living cells were observed using the confocal microscope in the epifluorescence mode [see Figure 2]. In the short time scale, an initial cytoplasmic localization of TPA (no labelling was detected at the nucleus level) was found. However, the fluorescence signal localization was dramatically modified under observation. Indeed, a strong decrease in the cytoplasmic fluorescence intensity concomitant to a strong increase in the nuclear fluorescence intensity was observed. Moreover, the transmission image showed a concomitant formation of membrane blebs, suggesting a photo-induced cell death.

*Conclusion:* TPAs clearly behave differently in fixed and living cells. They directly localize in the nucleus of fixed cells while they initially localize in the cytoplasm of living cells with a subsequent re-localization (from cytoplasm to nucleus) upon photoillumination.

### Example 3: Photoinduced membrane blebbing by TPAs and simultaneous subscellular relocalization: Insight into the mechanism of action of TPAs on living cells.

### Experiment 1: Epifluorescence microscopy

TPAs were incubated with HeLa for 2 hours at a concentration of 2 µM of TP3Bzim or TP2Py. Epifluorescence imaging *[Excitation filter: 450-490 nm; emission filter: 515-700 nm; irradiance, 1.8 W*/*cm²]* shows that the subcellular localization of TPAs (TP2Py in Fig. 2A or TP3Bzim in Fig. 2B) in living HeLa cells, at early time of observation was mainly cytoplasmic. The same result was found with living MCF-7 cells.

Due to its well marked emission in the red region of the spectrum, *i.e*. 634 nm, the subcellular localization of TP2Py at a concentration of 2 µM using MCF-7 cells was studied using green trackers.

A co-localization signal was evidenced thanks to the confocal microscopy with the MitoTracker Green FM that stains mitochondria (Fig. 3) *[Excitation wavelengths were 458 and 488 nm for TP2Py and MitoTracker Green FM, respectively; emission slit settings: 560-720 nm and 500-550 nm for TP2Py and MitoTracker Green FM, respectively].*

As shown in Fig. 2, the observation/excitation (after an incubation time of 2 hours) triggered a rapid re-localization of the fluorescence signal from mitochondria to the nucleus. This nuclear translocation began before t = 1 min and nuclear accumulation was optimal between 1 and 5 min (fluence or light dose, 108 J/cm²). In the transmission mode, the formation of membrane blebs was detected at t = 5 min and subsequent dramatic morphological changes (nucleus and cell shrinkages) were observed from t = 15 min, highly suggesting that cells undergo apoptosis.

All the above-mentioned observations done with Tp2Py (formula 21) and TP3Bzim (formula 21) were repeated with Tp3ox5Py (formula I), Tp2Bzim (formula 2I), Tp2Pyo (formula 21) and Cbz2Py (formula 21) (using 2 µM of TPA pre-incubated with living MCF-7 cells for 2 h before initial illumination under epifluorescence microscopy which triggers the cytoplasm -> nucleus re-localization).

The induction of cell death was clearly triggered by excitation light since:
- the initial observation consistently shows a mitochondrial localization of TP2Py, regardless of the incubation time (cells + TPA) preceding observation/illumination. For instance, the initial mitochondrial localization of TP2Py was also observed after 16-hour incubation (Fig. 4, left) in a similar manner than that observed after 2-hour incubation (Fig. 2A). A further nucleus translocation kinetics was observed after 15 min under continuous light illumination (Fig. 4, right showing the photo-induced re-localization of the fluorescence signal of TP2Py from the cytoplasm to the nucleus, 15 min after the beginning of irradiation of cells pre-incubated with TP2Py for 16 hours). *[Excitation filter, 450-490nm; emission filter, 515-700 nm; irradiance, 1.8W*/*cm²]. Conclusion:* the re-localization of TP2Py from the cytoplasm to the nucleus and the associated cell death actually depends on the illumination process, regardless of the pre-incubation time.
- Each time the illumination field was changed for a given coverslip, the overall process (nuclear translocation of TPA + membrane blebbing) restarted, regardless of the time period separating the incubation and observation (data not shown). Thus, only cells in the irradiated area undergo the apoptosis process. It is important to note that exposure to natural light did not trigger any of the associated phenomena described above (nuclear translocation, blebbing) under the same concentration condition (TPA, 2µM).

Altogether, these data suggest that both the presence of TPA and light illumination were required for inducing cell death. In the dark or exposed to natural light only, the TPA remained localized in mitochondria and did not appear to significantly perturb the cell morphology. The dark toxicity of TP2Py (2I) or TP3Bzim (2I) was negligible below 50µM in HeLa cells. In MCF-7 cells, the dark toxicity was slightly higher, however the TC₅₀ values (toxicity concentration 50%) were found to be above 30µM. No dark toxicity was evidenced with Tp3ox5Py (I) and Cbz2Py (21) up to 50 µM in MCF-7 cells; the Tp2Bzim (21) and Tp2Pyo (21) were found to be more toxic when tested in dark on MCF-7 cells with TC50 values of 10 and 20 µM, respectively.

### Experiment 2: confocal fluorescence microscopy

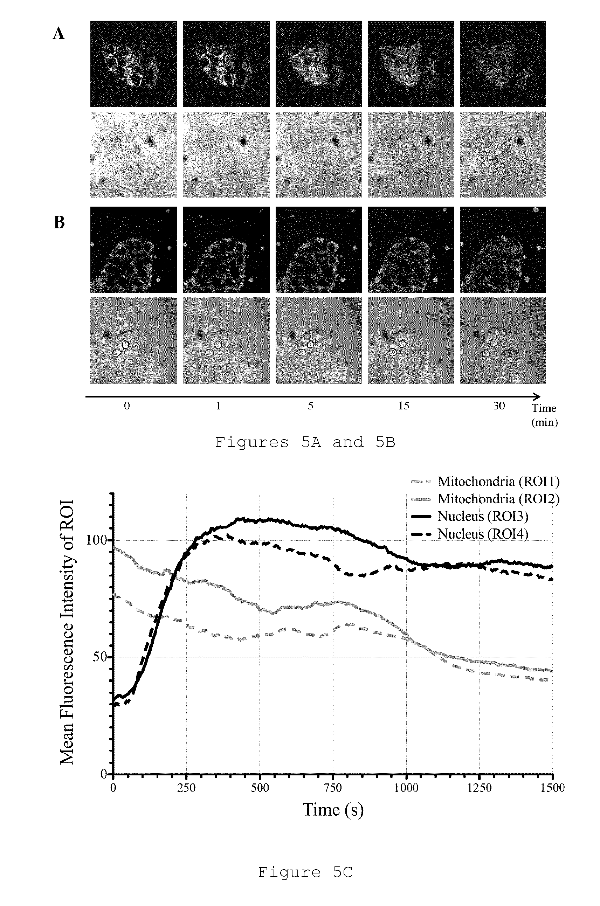

The photo-induced nuclear translocation and membrane blebbing were next assessed using confocal fluorescence microscopy [λ*_{exc}* = *458 nm; emission slit settings: 560-720nm and 530-690nm for TP2Py and TP3Bzim, respectively]* under experimental conditions similar to those described in experiment 1 for epifluorescence experiments. The corresponding transmission images illustrating the concomitant membrane blebbing are shown below the confocal fluorescence images. Living MCF7 cells were incubated with 2µM TP3Bzim or TP2Py for 2 hours before observation. As shown in Fig. 5A (TP2Py) and in Fig. 5B (TP3BZim), the results obtained with confocal imaging were similar to those obtained with epifluorescence imaging *(experiment 1),* with however, some discrepancies in the time courses of event appearance, most likely due to the power of the excitation source which is different between epifluorescence and confocal experiments (see below). Under light illumination, the fluorescence emission intensity of TP2Py continuously increased in the nucleus (after a lag period of approximately 2-4 min) to reach a maximum value at t = 8-10 min (Fig. 5C). In the same time, the fluorescence intensity in mitochondrial regions continuously decreased and then, was found to be anti-correlated to the fluorescence signal measured in the nucleus (the subsequent parallel decrease of both signals was related to photobleaching). Based on both criteria (re-localization of the fluorescence signal and the formation of blebs), the deduced fluences were 22 and 40 J/cm² for TP2Py and TP3Bzim, respectively.

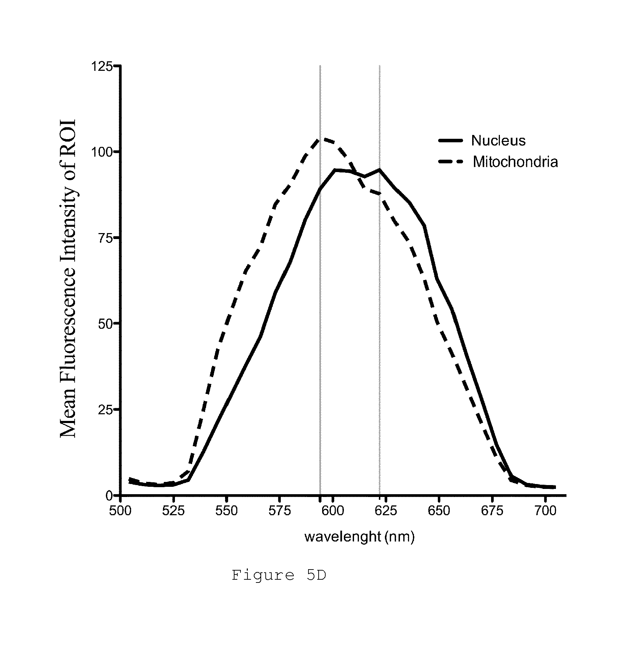

Interestingly, the fluorescence emission spectra of TP2Py in nuclei or in mitochondria were distinct (Fig. 5D) with a maximum of emission centered at 624 nm in nuclei, close to the one obtained *in vitro* for DNA-bound TP2Py (634 nm) *(*Allains et al. 2007 ChemBioChem 8, 1-11*)*. In contrast, the maximum of emission was blue-shifted in mitochondria (594 nm), suggesting that TP2Py probes (two-branches TPA compound) a distinct environment. In contrast to two-branch TPA compounds, the fluorescence of three-branch TPA (TP3Bzim) was found to be less sensitive to the environment, and consequently, the emission spectrum of TP3Bzim remained the same, regardless of its localization, nuclear or cytoplasmic (data not shown

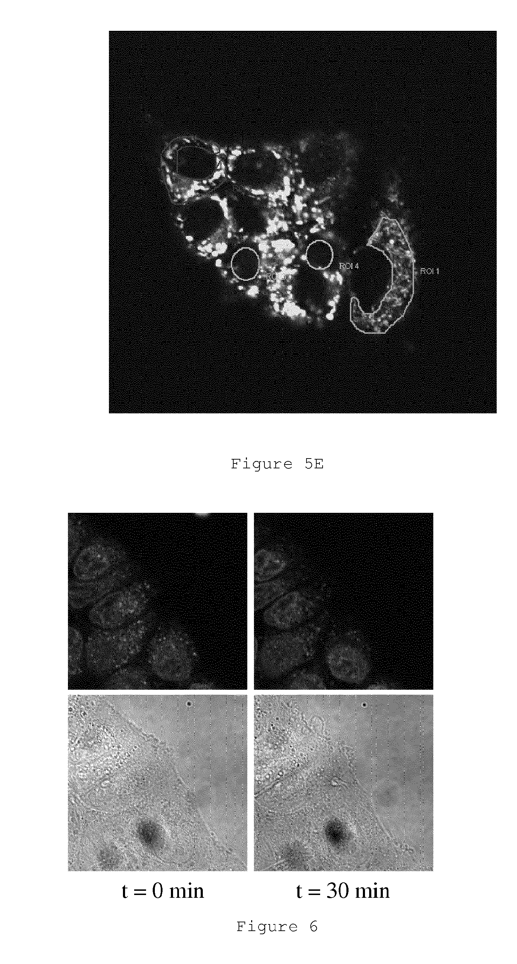

Figure 5E shows selected regions of interest in confocal fluorescence image (irradiance, 30 mW/cm²) used in temporal (Fig.5C) and spectral (Fig.5D) analysis of the fluorescence signal.

*Conclusion:* In the absence of illumination, TPAs are initially localized in the cell cytoplasm, regardless of the duration of the pre-incubation (cell + TPA; tested up to 16 hours). Only the TPA/light combination leads to cell death, concomitantly to the re-localization of TPA from cytoplasm to nucleus.

### Example 4: Apoptosis phenomenon linked to the cationic nature of the TPA derivatives

Living MCF-7 cells were treated with 2 µM a neutral TPA: TP3^{N}Bzim (see formula below) for two hours and observed by confocal fluorescence imaging with *[*λ*_{exc}* = *458nm]* at different times, t = 0 (left) and t = 30 min (right).

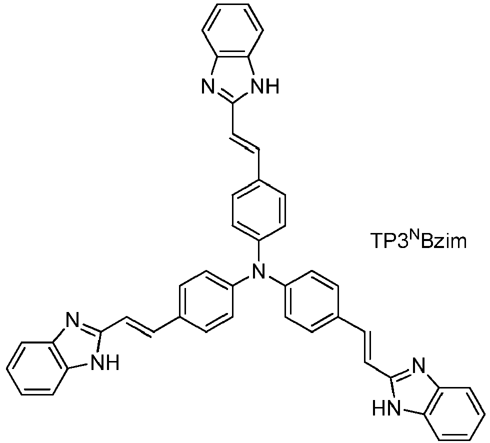

Figure 6 shows that the neutral TPA derivative (TP3^{N}Bzim) was found to be mainly localized in nuclei, even in early microscopic observation. Most importantly, no membrane blebbing or morphological changes of the cells was observed under light illumination (data not shown).

*Conclusion:* This result suggests that the cationic charge ensures a mitochondrial localization and prevents the nuclear localization (in absence of light illumination, and that the DNA binding / nuclear localization is not directly responsible for apoptosis occurring on short time scale.

### Example 5: Dose response and illumination power-dependencies of the TPA-mediated cell death under confocal observation

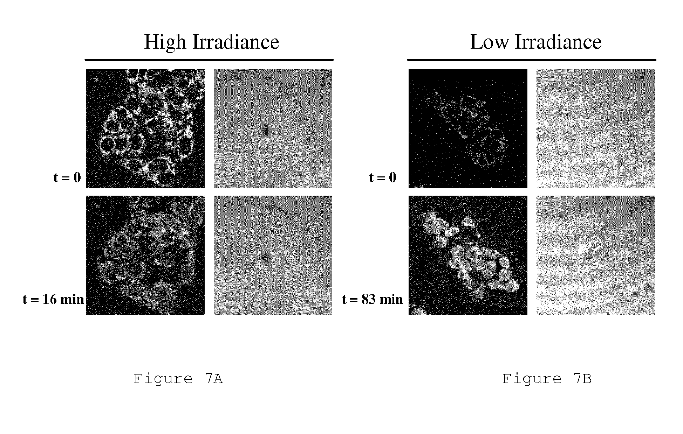

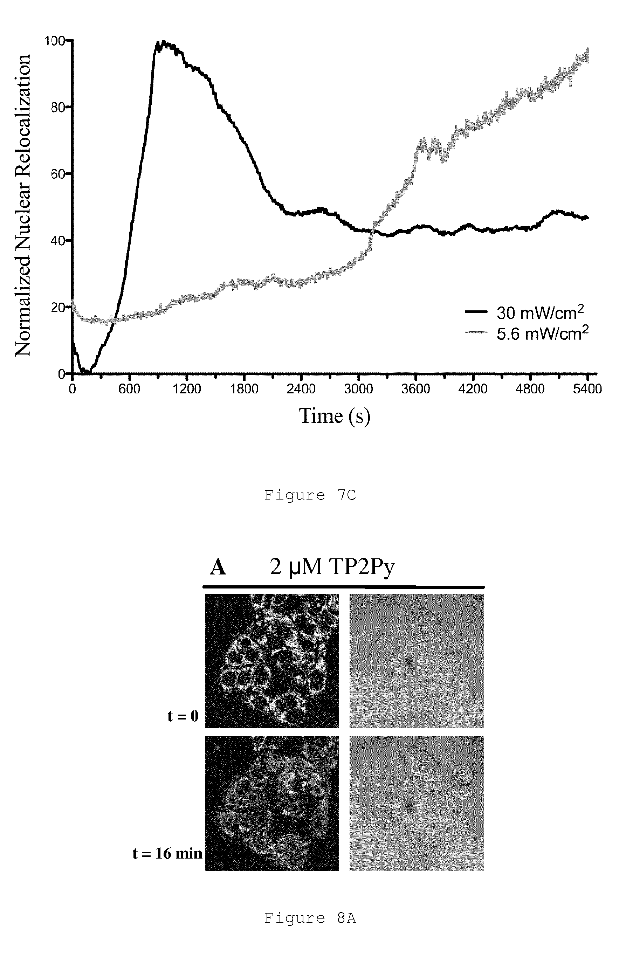

The TP2Py compound at a concentration of 2 µM was first incubated with living MCF-7 cells for 2 hours and then observed with confocal microscope with varying illumination power of the laser source *[λ=458 nm; emission slit setting: 560-720 nm].* Clearly, Fig.7 shows that the excitation power influences the time course of the nuclear translocation of TP2Py, with a re-localization starting at tₛₜₐᵣₜ = 4 min and peaking at tₘₐₓ = 15 min for an irradiance of 30 mW/cm² (Fig. 7A) while the same process was time-shifted for a lower excitation power for an irradiance of 5.6 mW/cm²: tₛₜₐᵣₜ = 50 min and tₘₐₓ = 1h22min) (Fig. 7B). For each condition, the fluorescence intensity in ROIs corresponding to the nucleus was plotted as a function of time (the initial time value, t = 0, represents the first observation) (FIG.7C). Using integration time based on tₘₐₓ values, light dose values, determined from experiments performed at irradiance of 5.6 or 30 mW/cm², were consistent (27.6 and 27 J/cm² respectively). These values are in accordance with the above-mentioned value for TP2Py (fluence, 22 J/cm² in Fig. 5).

*Conclusion:* The fluences characterizing TPAs correspond to non-invasive values.

### Example 6: Influence of the concentration of the TPA on the nuclear relocalization of TPA.

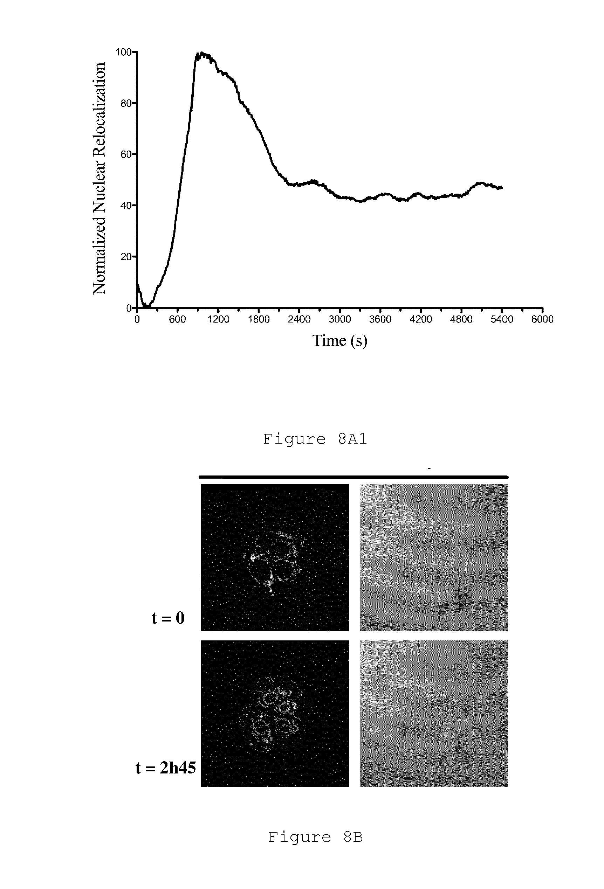

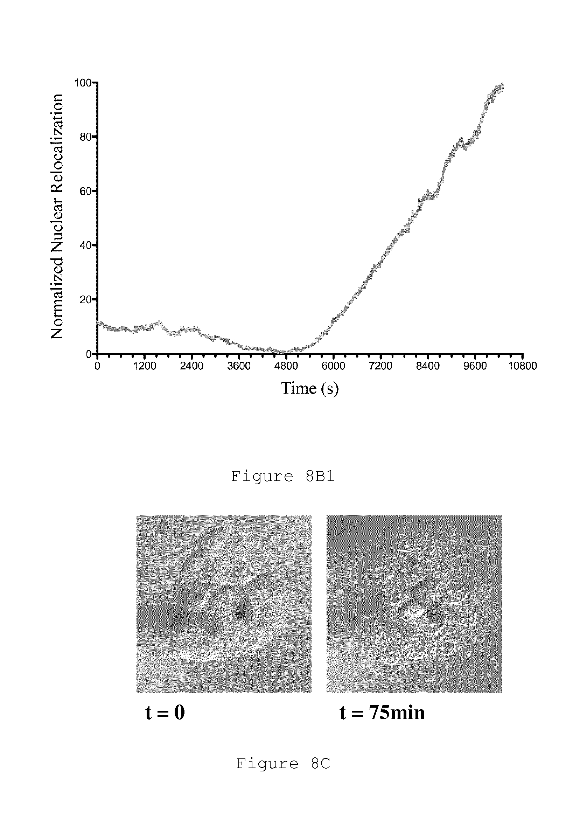

Living MCF-7 cells were treated with varying concentrations of TP2Py for two hours (2µM; 0.2µM and 0.02µM) and observed with a confocal microscope [λ*_{exc} = 458nm; irradiance 30mW*/*cm², emission slit setting: 560-720nm.]* (FIG 8A: 2µM; FIG 8B: 0.2µM; FIGBC: 0.02µM).

A clear dose response effect on the nuclear translocation time course was observed for a given excitation power (30 mW/cm²) (Fig. 8). Indeed, at 0.2µM TP2Py (Fig. 8B), the tₛₜₐᵣₜ and tₘₐₓ values were 1h15min and >2h, respectively (while at 2 µM (Fig. 8A), tₛₜₐᵣₜ = 4 min and tₘₐₓ = 15 min). The delay in the appearance of membrane blebbing parallels the delay in the re-localization event.

For experiments using 0.2 (FIG 8B1) and 2µM (FIG 8A1) TP2Py, the fluorescence intensity in ROIs corresponding to the nucleus was plotted as a function of time (the initial time value, t = 0, represents the first observation). As expected, the corresponding light dose value depends proportionally on the concentration of TP2Py used (27 J/cm² and 306 J/cm² for concentrations of 2 and 0.2µM TP2Py, respectively). Interestingly, even under conditions where the TP2Py concentration was too low for measuring its fluorescence signal (20nM), the membrane blebbing was observed under transmission mode. However the first blebs appeared at t = 75 min under constant light excitation (Fig. 8C).

*Conclusion:* The light illumination-dependent cell death may occur within a large range of concentration (from 20nM to 2µM), much below the toxicity concentration (see below). The time of exposure must be adapted to each TPA concentration.

### Example 7: Measurement of the cytophotoxicity by MTT assay in two cell lines (HeLa and MCF-7 cells)

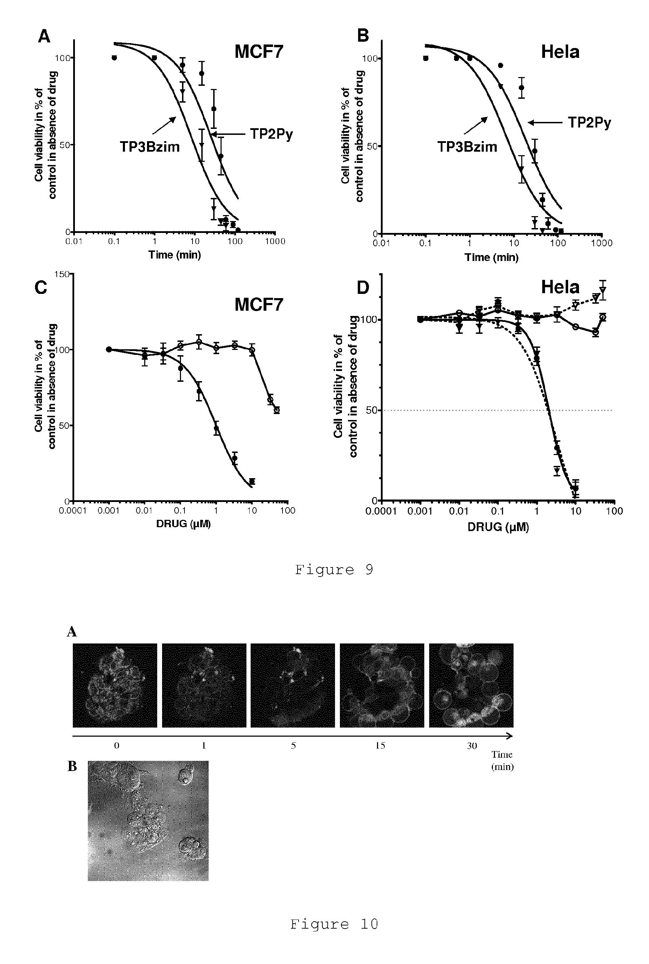

Cell viability (in % of control treated in the absence of TPA) as a function of time illumination (Fig.9A and Fig.9B) for a given TPA concentration (2µM) or as a function of TPA concentration (for a given time illumination) (Fig.9C and Fig.9D) has been assessed. Different experiments were conducted and described below:
- MCF7 cells were treated with 2µM of TP2Py or TP3Bzim and exposed to illumination (Fig.9A);
- HeLa cells were treated with 2µM of TP2Py or TP3Bzim and exposed to illumination (Fig.9B);
- MCF7 cells were treated with TP2Py, 40 min illumination (black circles) or without illumination (white circles) (Fig.9C)
- HeLa cells were treated with TP2Py [30 min illumination (black circles) or without illumination (white circles)] or TP3Bzim [15 min illumination (black triangles) or without illumination (white triangles)]

For each experiment, the source of excitation was a Mercury lamp (130W; 380-600nm) with an excitation filter centered at 452nm (± 45 nm), irradiance, 60mW/cm².

First, the experiment was calibrated by determining the time of illumination exposure which leads to the toxicity 50%, for a given concentration of TPA (2 µM) and for an irradiance of 60mW/cm². As shown in Fig. 9A and 9B for MCF-7 and HeLa cells, respectively, the corresponding times for TP2Py and TP3Bzim were significantly different. This characteristic time was then used for the plots shown in Fig. 9C and 9D which represent the cell viability as a function of TPA concentration (in HeLa and MCF-7 cells, respectively). The dose responses as shown in Fig. 9C & 9D indicate that TP2Py and TP3Bzim display only moderate toxicity in the absence of illumination (dark toxicity). The dark toxicity of TP2Py or TP3Bzim was negligible below 50µM in HeLa cells (Fig. 9D). In MCF-7 cells, the dark toxicity was higher (the TC₅₀ value of TP2Py was found to be above 30µM) (Fig. 9C). As shown in HeLa cells, TP2Py exhibits more toxicity than TP3Bzim. Moreover, TP3Bzim is more efficient for the photo-induced apoptosis because the same TC₅₀ under illumination (toxicity concentration 50%) requires an illumination time of 15 min for TP3Bzim and 30 min for TP2Py (corresponding light dose values: 54 and 108 J/cm², respectively). Regarding light-induced toxicity, the responses of HeLa and MCF-7 cells to TP3Bzim and TP2Py compounds are similar. Finally, exposure to natural light was not able to induce apoptosis of HeLa or MCF-7 cells, using a standard concentration of 2µM. However, both blebbing and re-localization of the fluorescence signal to the nucleus after 2-hour incubation of 30-50µM TPA with HeLa cells under natural light was observed (data not shown).

*Conclusion:* This result suggests that (i) the toxicity of TPA under natural light or the dark toxicity are moderate and that (ii) the observed TPA-mediated effects of natural light requires very high concentrations of TPAs (above 30µM).

### Example 8: cellular death induced by a two-photon excitation of TPAs

Living MCF-7 cells were treated with 2 µM of TP2Py for 2 hours, exposed to two-photon excitation and simultaneously observed by multiphoton microscopy *[using- a pulsed IR laser source for excitation Mai Tai, Spectra Physics,* λ*ₑₓ* = *860nm;* irradiance, 1.25W/cm², *emission slit setting: 560-720nm].* This wavelength represents the best compromise for optimizing the two-photon fluorescence signal and minimizing photobleaching, because the susceptibility to photobleaching was found to be greater under two-photon excitation (photobleaching was even much greater using λₑₓ = 900-920nm). As shown in Fig. 10, the two-photon excitation of TP2Py lead to a re-localization of the TPA compound into the nucleus of living MCF-7 cells, starting at t = 5-15 min after the first excitation, (light dose ≈ 300 J/cm²) with a visible associated blebbing in fluorescence images (Fig. 10A); the blebbing phenomenon was also observed in the transmission mode as shown in Fig. 10B, corresponding to t = 30 min.

*Conclusion:* TPAs are compatible with photo-induced cell death via a 2-photon excitation process.

### Example 9: Evidences for an apoptosis-mediated mechanism of cell death induced by TPAs.

### By PARP detection

HeLa cells were plated in 6-well plates (4.10⁵ cells/well) and incubated with 2 µM TP2Py for 2h at 37°C. After varying illumination times (up to 90min) at 17 mW.cm⁻² *(Mercury lamp (130W; 380-600nm) + excitation filter centered at 452 nm (± 45 nm)),* cells were collected by scraping, washed once in ice-cold PBS, treated with lysis buffer (1% Nonidet P-40, 20 mM Tris pH 8.0, 137 mM NaCl, 10% glycerol (v/v)) supplemented with 1 mM phenyl-methylsulfonyl fluoride, 1 mM sodium orthovanadate and anti-protease cocktail (Complete, Boehringer Mannheim, Germany) for 20 min on ice and further centrifuged at 10000g for 10 min. Protein concentrations were determined using the bicinchoninic acid method (micro BCA, Pierce, USA). 40µg of detergent soluble proteins were separated by SDS-PAGE under reducing conditions followed by Western blotting. Membranes were first incubated overnight at 4°C with the rabbit primary polyclonal antibody anti-PARP (Cell Signaling Technology). Membranes were then probed with horseradish peroxidase-labelled anti-rabbit IgG (dilution, 1/2000) (Dakocytomation). Detection was based on a chemiluminescence detection system (GE Healthcare, Orsay, France). The immunodetection of beta-actin (supposed to be constant regarding its concentration, regardless of the experimental condition) was used as an internal loading control.

It is known that nuclear DNA repair enzyme PARP (poly-ADP ribose polymerase) is a target of caspase 3, and its cleavage can serve as a biochemical marker of apoptosis *(*Kuznetsov et al. 2011 Cancer Res. 64, 5760-66; Sarveswaran et al. 2010 Cancer Let. 291, 167-76*;* Lee et al. 2011 Molecular Cell 43, 180-91*).*

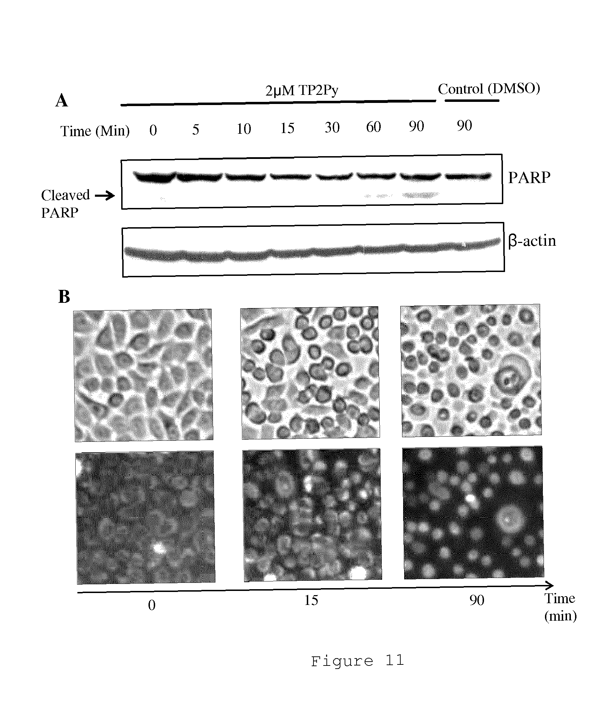

The cleavage of PARP, a hallmark of apoptosis, was assessed by Western blot using an anti-PARP antibody that recognizes both the entire and cleaved PARP (Fig. 11).

As shown in Fig. 11A, PARP cleavage was detected after 60 min illumination and its intensity was dependent on the illumination time. No cleavage was evidenced in the control (not treated with TP2Py but only with DMSO (1% v/v)), after 90 min illumination. The concomitant alteration of the cell morphology induced by TP2Py and light illumination was observed before the detection of PARP cleaved products (Fig. 11B). Indeed, after 2 hour incubation with TP2Py but without any further light illumination, the HeLa cells displayed a normal morphology as observed using transmission or epifluorescence microscopy (Fig. 11B, left); after 15 min illumination, a few cells were detached (Fig. 11B, middle), whereas all cells displayed morphological alteration, typically observed during apoptosis, after 90 min illumination (Fig. 11B, right).

### By Annexin V staining

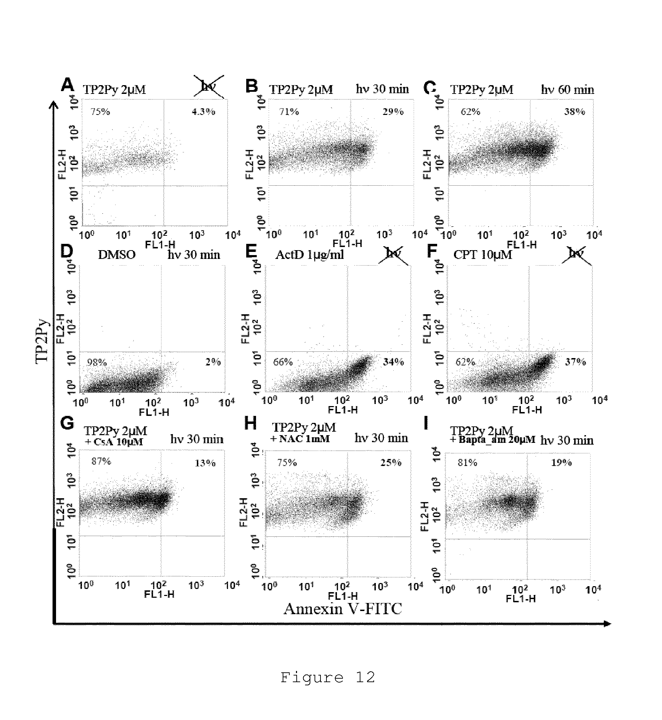

MCF-7 cells were plated in 12-well plates (2.10⁵ cells/well) and incubated with 2µM of TP2Py for 2h at 37°C. Under specific conditions (see Fig. 12), cells were further incubated with NAC (1mM) for 30 min, Bapta-AM (20µM) for 3h, or CsA (10µM) for 1h at 37°C. After a time illumination of either 30 or 60 min at 40 MW.cm⁻² *(Mercury lamp (130W; 380-600nm) + excitation filter centered at 452nm (± 45nm)),* cells were gently scraped and pooled with medium floating cells. Cells were individualized in PBS-EDTA (10mM) and stained as described by the manufacturer (Roche, Germany). Briefly, the cell pellet was re-suspended in 500µl Annexin V-HEPES solution (10 mM HEPES-NaOH, pH 7.4, 140mM NaCl, 5mM CaCl2) and incubated on ice for 30 min in the dark. Cells were then washed in ice-cold HEPES buffer before analysis. Fluorescence signals of fluorescently labelled (FITC) Annexin V and TP2Py were measured by a FACSCalibur flow cytometer (Becton-Dickinson, Sunnyvale, CA, USA) using FL1 and FL2 detectors, respectively.

Different experiments are summarized below:
- MCF-7 cells treated with 2µM TP2Py for 2 hours and then stained with FITC-conjugated Annexin V immediately (Fig.12A) without light exposure;
- MCF-7 cells treated with 2µM TP2Py for 2 hours and then stained with FITC-conjugated Annexin V after a time illumination (Exc, 452 nm) of 30 min (Fig.12B);
- MCF-7 cells treated with 2µM TP2Py for 2 hours and then stained with FITC-conjugated Annexin V after a time illumination (Exc, 452 nm) of 60 min (Fig.12C);
- control MCF-7 cells treated with DMSO (1%) only and illuminated for 30 min before Annexin V staining (Fig.12D);
- control MCF-7 cells treated with 1µg/mL actinomycin D (ActD), no illumination before Annexin V staining (Fig.12E);
- control MCF-7 cells treated with camptothecin (CPT, 10µM)) (Fig.12F) for 2.5 hours, no illumination before Annexin V staining;
- MCF-7 cells treated during 2 hours with 2µM TP2Py and 10µM cyclosporine A (CsA) and illuminated for 30 min before Annexin V staining (Fig.12G);
- MCF-7 cells treated during 2 hours with 2µM TP2Py and 1 mM N-Acetyl cysteine (NAC) and illuminated for 30 min before Annexin V staining (Fig.12H); and
- MCF-7 cells treated during 2 hours with 2µM TP2Py and 20µM Bapta-am (Fig.12I) and illuminated for 30 min before Annexin V staining.

The horizontal (FL1-H) and vertical (FL2-H) axes correspond to the fluorescence signal of Annexin V and TP2Py, respectively. The percentage of population with a high mean fluorescence signal is indicated in the corresponding quadrant (top right or bottom right in the presence or absence of TP2Py, respectively).

In this example, apoptosis was then assessed by FITC-conjugated Annexin V staining and flow cytometric analysis (Fig. 12), probing the externalization of phosphatidyl serine (Lim et al. 2009 British J. Cancer 101, 1606-12*;* Sarveswaran et al. 2010 Cancer Let. 291, 167-76). In cells treated with TP2Py without light illumination (Fig. 12-A) or in the control experiment (Fig. 12-D: DMSO only and 30 min of light illumination), 4.3% (Annexin V positive, TP2Py positive) and 2% (Annexin V positive, TP2Py negative) of cells displayed a high mean fluorescence intensity (HMFI), respectively. The HMFI population (Annexin V positive, TP2Py negative) was significantly higher when cells were treated with the proapoptotic compound actinomycin D (Fig.12-E; HMFI = 34%) or camptothecin (Fig. 12-F; HMFI = 37%). Upon light illumination of TP2Py-treated cells, the HMFI populations (Annexin V positive, TP2Py positive) also significantly increased, *i.e*. 29% and 38% after 30 min and 60 min illumination, respectively, confirming that TP2Py induced apoptosis in a light-dependent manner (Fig. 12-B-C). The apoptosis is related to mitochondria since CsA (cyclosporin A), a compound that inhibits mitochondrial permeabilization, prevented the TP2Py-mediated photo-induced apoptosis (Fig. 12-G; HMFI = 13%). Moreover, the TP2Py-dependent apoptosis was also protected by antioxidant NAC (N-Acetyl cysteine) (Fig. 12-H; HMFI = 25%) or the intracellular calcium chelator Bapta-am (1,2-bis(o-Aminophenoxy)ethane-N,N,N',N'-tetraacetic Acid Tetra(acetoxymethyl) Ester) (Fig. 12-1; HMFI = 19%), showing that the TP2Py-mediated photo-induced apoptosis phenomenon is related to both oxidative and calcium deregulation processes. It has been confirmed that TP2Py causes apoptosis of MCF-7 cells in a light illumination-dependent manner.

*Conclusion:* The cell death process induced by the TPA/light combination is related to apoptosis.

### Example 10: Participation of the ROS (reactive oxygen species) in the TPA-mediated apoptosis

In order to assess the participation of ROS (reactive oxygen species) in the TPA-mediated apoptosis, a flow cytometric analysis was performed using a H₂DCF-DA probe which is a detector of ROS.

### Fluorescence detection of ROS generation:

CF-7 cells were plated in 12-well plates (2.10⁵ cells/well) and incubated with 2µM of TP2Py for 2h at 37°C. After a time illumination of 30 min *[Irradiance, 40mW*/*cm². The source of excitation was a Mercury lamp (130 W; 380-600 nm) with an excitation filter centered at 452 nm (± 45 nm)],* cells were gently scraped, individualized in PBS-EDTA (10 mM) and stained with 10µM of 5-(and-6)-carboxy-2',7'-dichlorofluorescein (H2DCF-DA) (Molecular Probe, USA) for 45 min. Cells were then washed in ice-cold HEPES buffer before analysis. Fluorescence signals of H2DCF-DA and TP2Py were measured by FACSCalibur flow cytometer using FL1 and FL2 detectors, respectively.

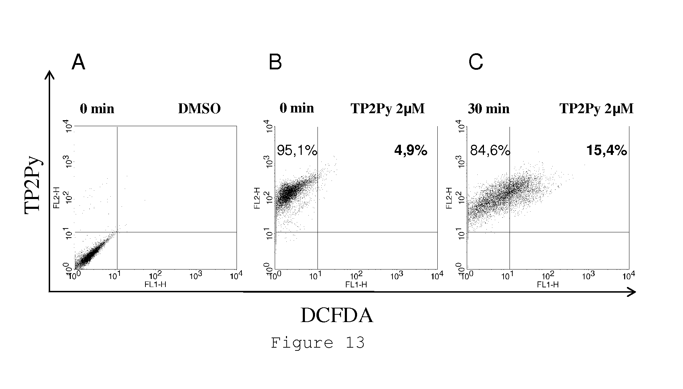

Different experiments are summarized below:
- Control MCF7 cells were treated with DMSO (1%) (Fig.13A) without light exposure;
- MCF7 cells were plated and treated with H₂DCF-DA and 2µM TP2Py for 2 hours without light exposure (Fig.13B);
- MCF7 cells were plated and treated with H₂DCF-DA and 2µM TP2Py for 2 hours after a time illumination of 30 min (Fig.13C)

The horizontal (FL1-H) and vertical (FL2-H) axes correspond to fluorescence signals of H₂DCF-DA and TP2Py, respectively.

As shown in Fig. 13, upon light illumination of TP2Py-treated cells, the HMFI populations (H₂DCF-DA positive, TP2Py positive) significantly increased from 4.9% to 15.4%, demonstrating that ROS mediate the TPA-dependent and light-dependent apoptosis phenomenon.

*Conclusion:* The apoptosis phenomenon induced by the TPA/light combination originates from a burst of the intracellular concentration of reactive oxygen species.

## Claims

1. Cationic triphenylamine derivative presenting the following general formula (I): 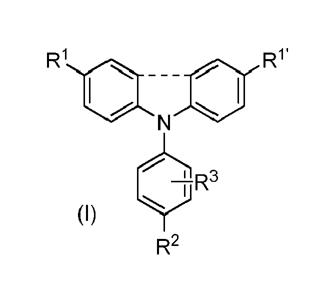 where
---- is an optional covalent bond;
each of R¹, R^{1'}, R² is the same or different and represents 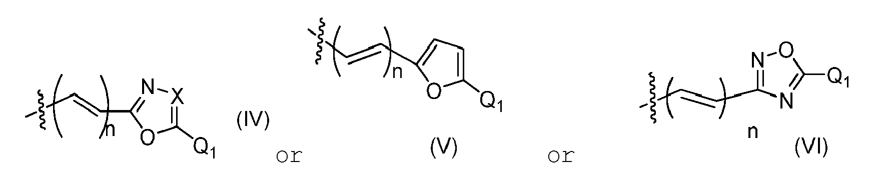 with X= CH or N, n=0 or 1 and Q₁ being :
- an heterocyclic group of formula (i) 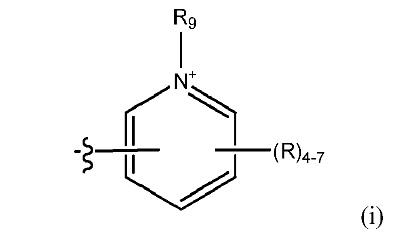 where R₉ represents an hydrocarbon group;
R₄ to R₇ represent independently an hydrogen or an hydrocarbon group;
- an heterocyclic group of formula (ii), (iii), (iv) or (v) 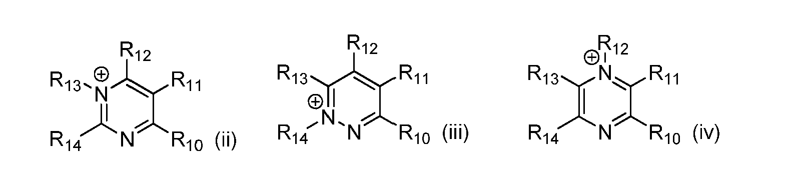 where R₁₀ represents a covalent bond linking said heterocyclic group of formula (ii), (iii) or (iv); R₁₁-R₁₄ represent independently of each other an hydrogen or an hydrocarbon group; 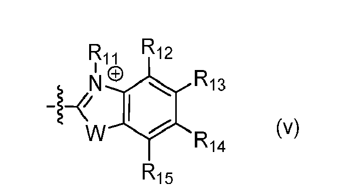 where R₁₁-R₁₅ each represents independently of each other an hydrogen or an hydrocarbon group; W represents an oxygen or NR₁₆ with R₁₆ being an hydrocarbon group;
R² can also be an hydrogen or a linking group which is a functional group capable of allowing the grafting, by a chemical reaction, of the compound of formula (I) on a biomolecule, or a hydrocarbon group comprising such a functional group;
R³ represents H or a linking group which is a functional group capable of allowing the grafting, by a chemical reaction, of the compound of formula (I) on a biomolecule, or a hydrocarbon group comprising such a functional group; with the proviso that compound (I) does not represent

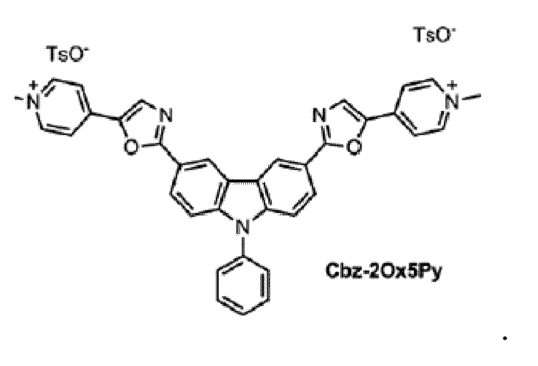

2. Cationic triphenylamine derivative according to claim 1, wherein at least two of R¹, R^{1'} or R² are identical.

3. Cationic triphenylamine derivative according to claim 2, wherein at least two of R¹, R^{1'} or R² represent (IV) with Q₁ being (i) or (v).

4. Cationic triphenylamine derivative according to claim 3, wherein W is NR₁₆ and R₁₂-R₁₅ are identical and represent H.

5. Cationic triphenylamine derivative according to anyone of claims 1-4 being a compound chosen in the group consisting of TP-2(Ox5Q1) and TP-3(Ox5Q1) 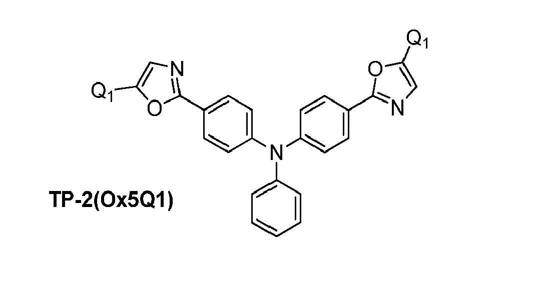 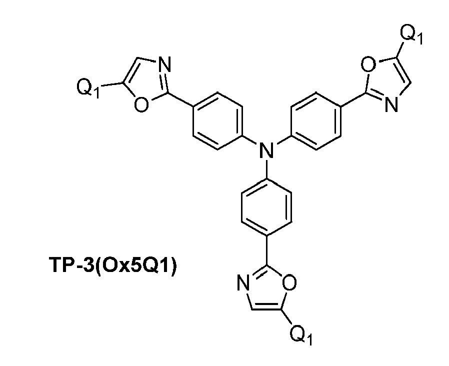 with

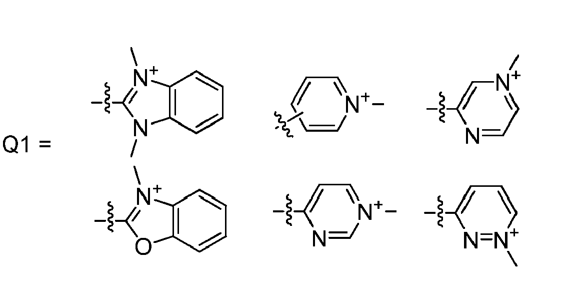

6. Cationic triphenylamine derivative according to anyone of claims 1-5 being a compound chosen in the group consisting of TP30x5Py (formula a), TP20x5Py (formula b), TP30x5BZim (formula c) and TP20x5BZim (formula d)

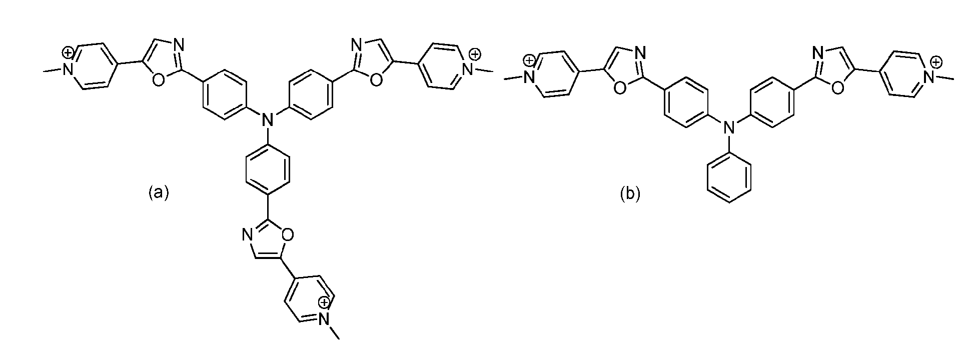

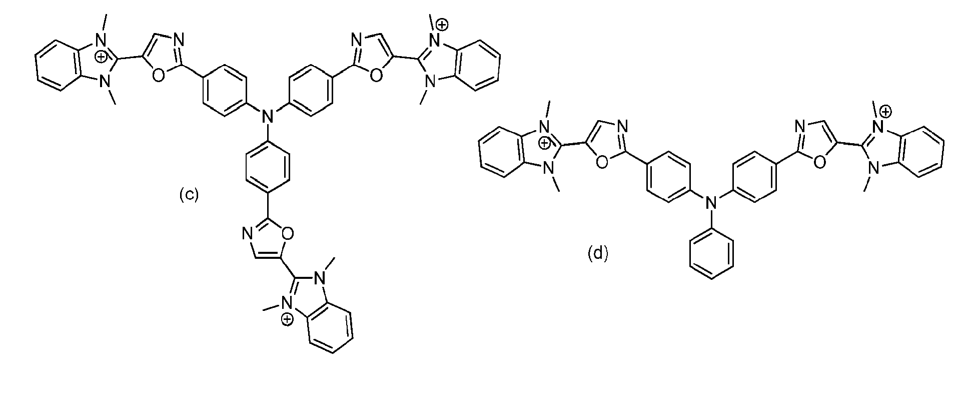

7. Process for preparing compound TP-2(Ox5Q1) or TP-3(Ox5Q1) as defined in claim 5 according to the following scheme 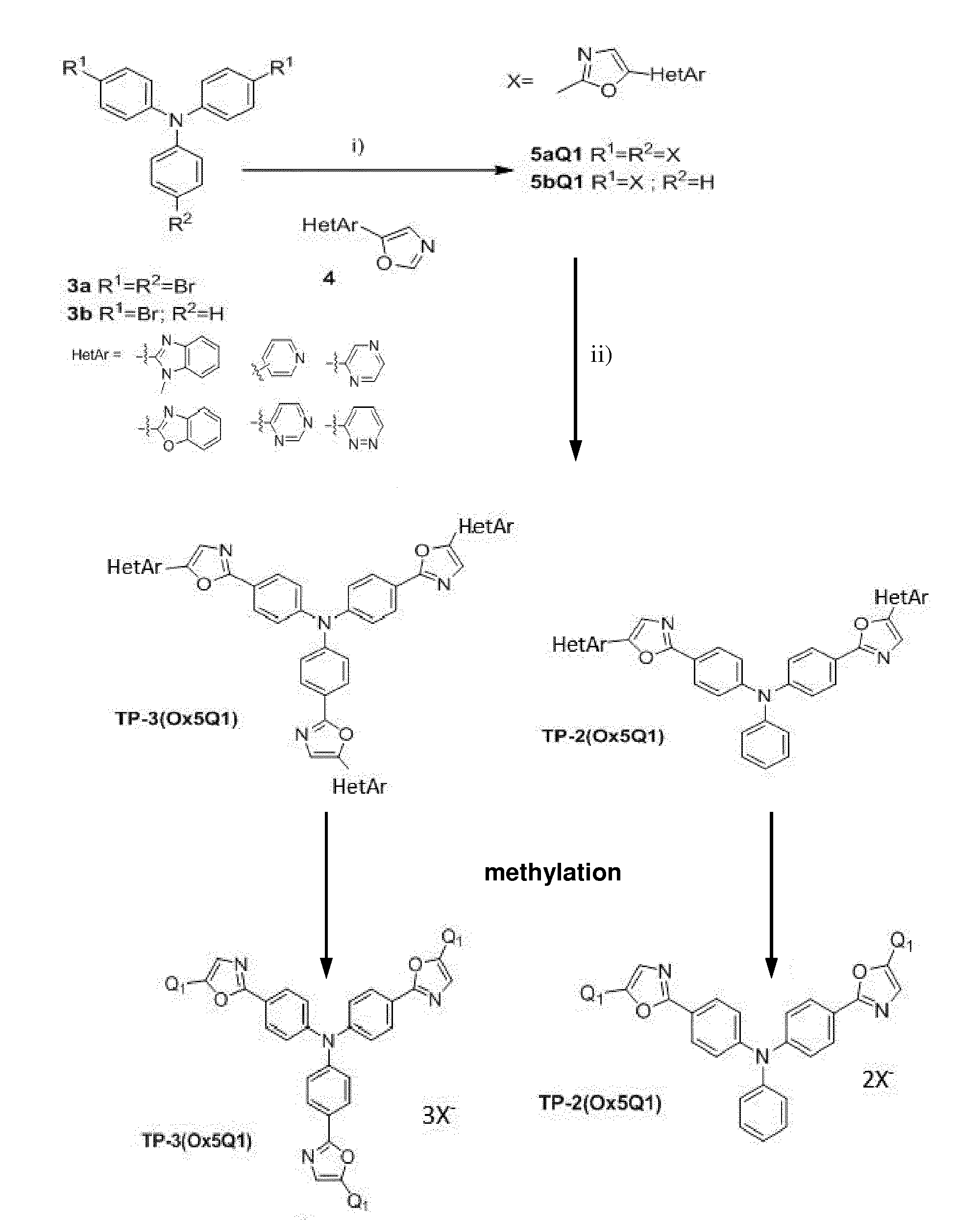 With 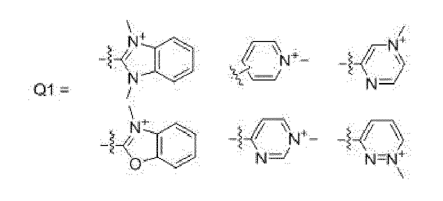 and X⁻ being a counter-ion

8. Cationic triphenylamine derivative according to anyone of claims 1-6 exhibiting a fluence ranging from 10 to 300 J/cm² and a fluence ranging from 50 to 500 J/cm² for triggering death cell by one photon excitation and two photons excitation, respectively.

9. A solution comprising a cationic triphenylamine derivative according to anyone of claims 1-6 and a solvent.

10. A pharmaceutical composition comprising a cationic triphenylamine derivative selected from the group consisting of compounds of formula (21) defined below, compounds of formula (I) defined above and mixture(s) thereof and a pharmaceutically acceptable carrier 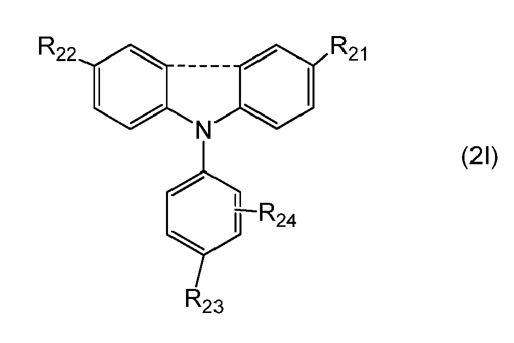 in which:
----- represents an optional covalent bond;
R₂₄ represents a hydrogen atom or a linking group, in which case:
1) if R₂₄ represents a hydrogen atom, then: R₂₁ represents a linking group or a group of formula (II) below: 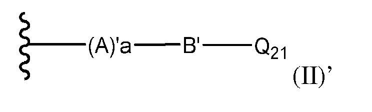 in which:
Q₂₁ represents:
a heterocyclic group of formula (i)' below: 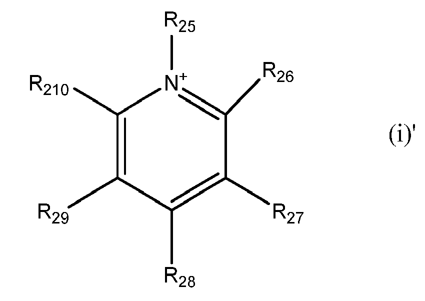
in which R₂₅ represents a hydrocarbon group; any one of R₂₆-R₂₉ and R₂₁₀ represents a covalent bond linking the said heterocyclic group to B, while the others from R₂₆-R₂₉ and R₂₁₀ represents, independently of each other, a hydrogen atom or a hydrocarbon group; or
a heterocyclic group of formula (ii)' or (iii)' below: 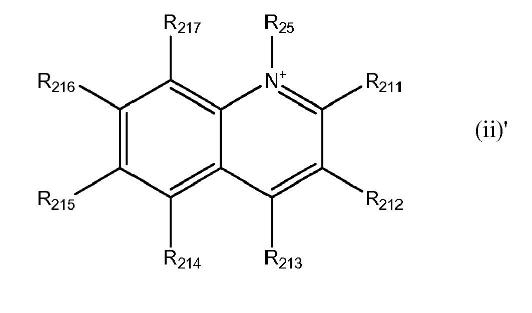 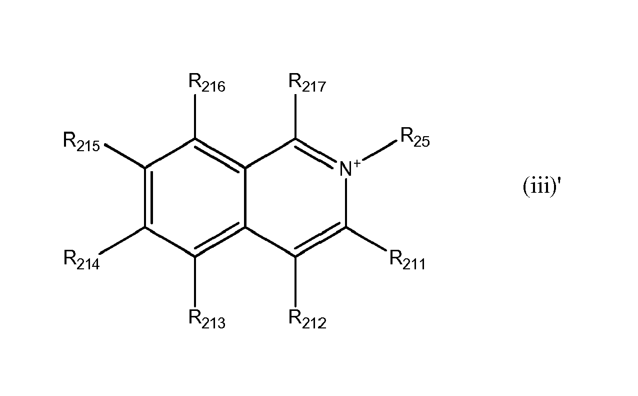
in which R₂₅ represents a hydrocarbon group; any one of R₂₁₁ to R₂₁₇ represents a covalent bond linking the said heterocyclic group to B, while the others from R₂₁₁ to R₂₁₇ represent, independently of each other, a hydrogen atom or a hydrocarbon group, it being possible for R₂₁₂, R₂₁₄ and R₂₁₆ to also form, respectively with R₂₁₁ and/or R₂₁₃, R₂₁₃ and/or R₂₁₅, and with R₂₁₅ and/or R₂₁₇, a bridging group; or
a heterocyclic group of formula (iv)' below: 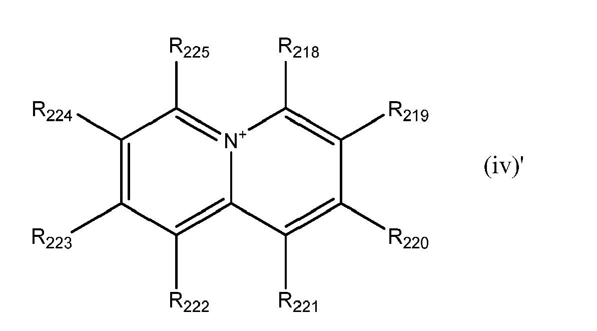
in which any one of R₂₁₈ to R₂₂₅ represents a covalent bond linking the said heterocyclic group to B, while the others from R₂₁₈ to R₂₂₅ represent, independently of each other, a hydrogen atom or a hydrocarbon group, it being possible for R₂₁₉, R₂₂₁, R₂₂₃ and R₂₂₅ to also form, respectively with R₂₁₈ and/or R₂₂₀, R₂₂₀ and/or R₂₂₂, R₂₂₂ and/or R₂₂₄, and with R₂₂₄ and/or R₂₁₈, a bridging group; or
a heterocyclic group of formula (v)' below: 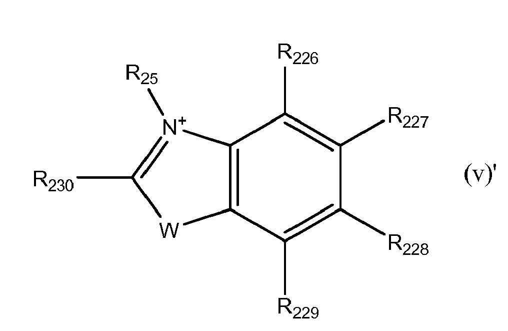
in which R₂₂₅ represents a hydrocarbon group; W represents an oxygen or sulphur atom or a group -N(R₂₃₁)- in which R₂₃₁ is a hydrogen atom or a hydrocarbon group, or a group - C(R₂₃₁)(R₂₃₂)- in which R₂₃₁ and R₂₃₂ are, independently of each other, a hydrogen atom or a hydrocarbon group; R₂₃₀ represents a covalent bond linking the said heterocyclic group to B, while R₂₂₆ to R₂₂₉ represent, independently of each other, a hydrogen atom or a hydrocarbon group, it being possible for R₂₂₇ to also form with R₂₂₆ and/or R₂₂₈ a bridging group, it being possible for R₂₂₈ itself to form with R₂₂₉ a bridging group;
a is equal to 0 (in which case A' is absent) or 1 (in which case A' is present);
A' and B' represent the groups below: 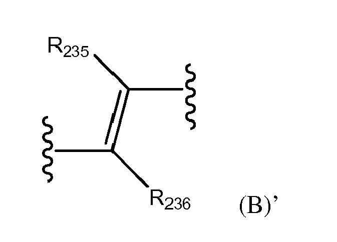 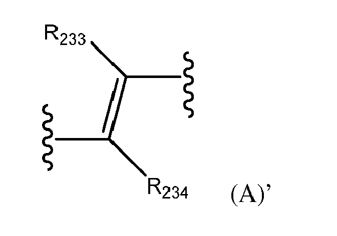
in which R₂₃₃ to R₂₃₆ represent, independently of each other, a hydrogen atom or a hydrocarbon group, it being possible for each of R₂₃₃ and R₂₃₆ to form with R₂₃₄ and/or R₂₃₅ a bridging group;
when R₂₁ represents a group of formula (II)' above, then R₂₂ also represents a group of formula (II)' above while, when R₂₁ represents a linking group, then R₂₂ represents a group of formula (III)' below: 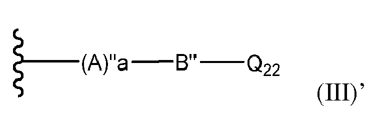 (III)'
in which:
Q₂₂ represents:
a heterocyclic group corresponding to any one of the formulae (i)' to (v)' above; or
a heterocyclic group of formula (vi)' below: 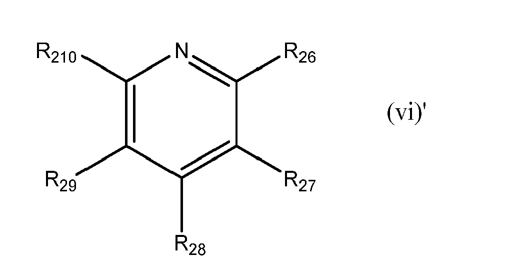
in which R₂₆-R₂₉ and R₂₁₀ have the same meaning as in the formula (i)' above; or
a heterocyclic group of formula (vii)' or (viii)' below: 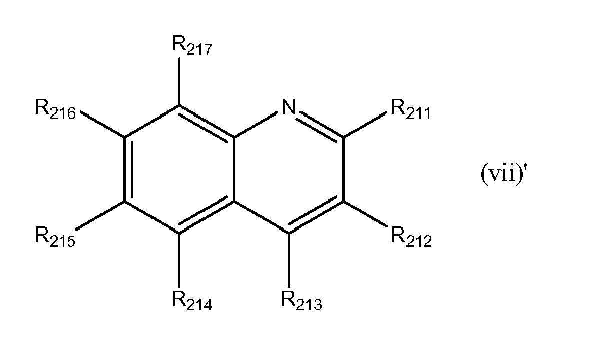 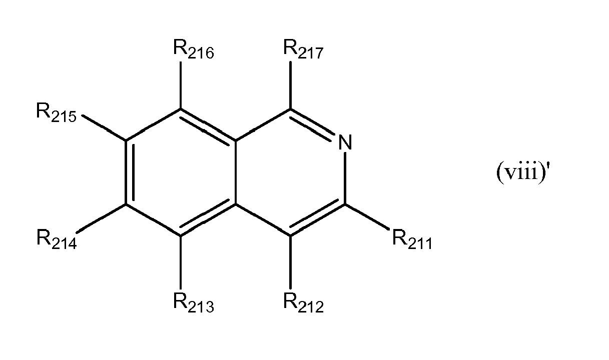
in which R₂₁₁ to R₂₁₇ have the same meaning as in the formulae (ii)' and (iii)' above; or
a heterocyclic group of formula (ix)' below: 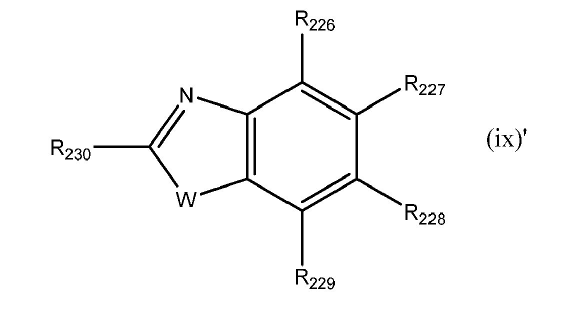
in which W, R₂₂₆ to R₂₃₀ have the same meaning as in the formula (v)' above;
a, A" and B" have the same meaning as above;
when R₂₁ represents a linking group, then R₂₃ represents a group of formula (III)' above while, when R₂₁ represents a group of formula (II)' above, then R₂₃ represents a hydrogen or halogen atom, a hydrocarbon group or a group of formula (II)' above;
2) if R₂₄ represents a linking group, then R₂₁ and R₂₂ represent a group of formula (III)' above while R₂₃ represents a hydrogen or halogen atom, a hydrocarbon group or a group of formula (III)' above;
in which the linking group is a functional group capable of allowing the grafting, by a chemical reaction, of the compound on a biomolecule, or a hydrocarbon group comprising such a functional group, and in which each of the abovementioned hydrocarbon groups may be substituted with one or more substituents, which are identical or different, and comprise one or more heteroatoms.

11. A pharmaceutical composition according to claim 10 wherein the cationic triphenylamine derivative is selected in the group consisting of compounds TP-2(Ox5Q1) and TP-3(Ox5Q1), preferably TP30x5Py, TP20x5Py, TP30x5Bzim, TP20x5Bzim, or in the group consisting of compounds TP2BZim, TP3BZim, TP3Pyo, TP2Pyo, Cbz2Py, Cbz3Py, TP2BZim, TP3BZim, TP2Py, TP3Py and mixture thereof and a pharmaceutical acceptable carrier.

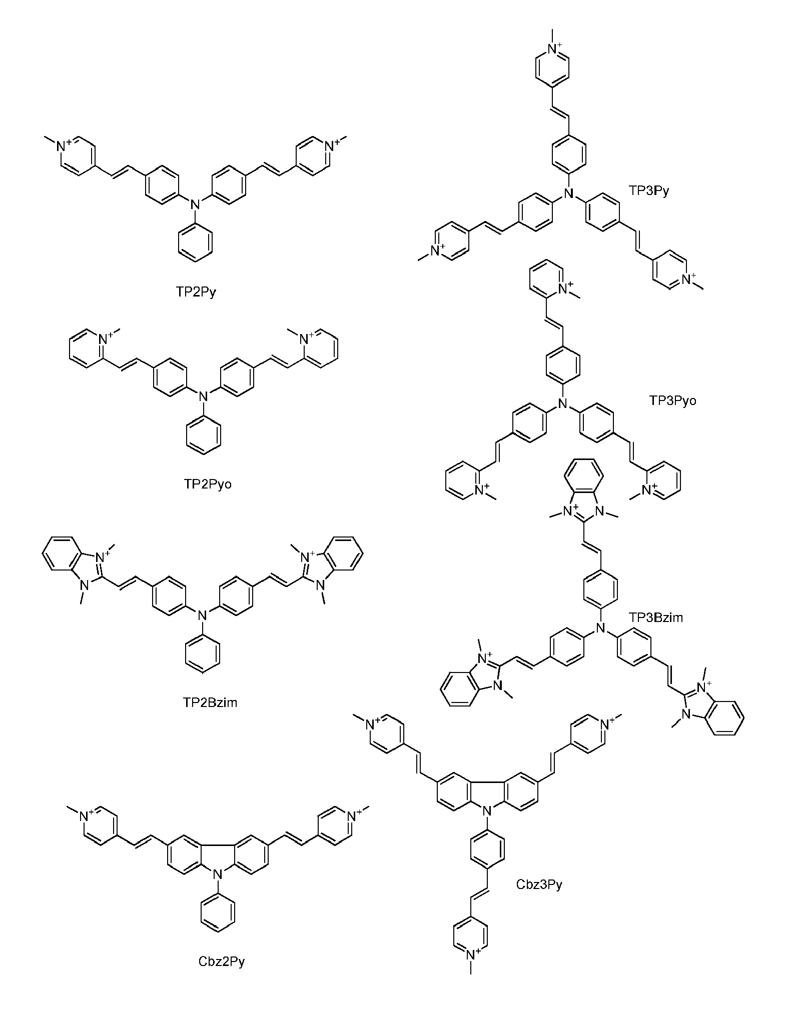

12. Cationic triphenylamine derivative of formula (I) or (21) or composition comprising said cationic triphenylamine derivative as defined in anyone of claims 10 or 11 for use as a drug.

13. Use of cationic triphenylamine derivative of formula (I) or (21) or of a composition comprising said cationic triphenylamine derivative as defined in anyone of claims 10 or 11 as photo active agent in dynamic phototherapy.

14. Use of cationic triphenylamine derivative of formula (I) or (21) or of a composition comprising said cationic triphenylamine derivative as defined in anyone of claims 10 or 11 as photo-dependent inducers of apoptosis on living cells.

15. Cationic triphenylamine derivative of formula (I) or (21) or composition comprising said cationic triphenylamine derivative as defined in anyone of claims 10 or 11 for use in the treatment of cancer, preferably by dynamic phototherapy.

16. Cationic triphenylamine derivative of formula (I) or (21) or composition comprising said cationic triphenylamine derivative as defined in anyone of claims 10 or 11 for use for treating cancer, such as:
- cervical cancer,
- breast cancer,
- skin cancer, especially in the treatment of non-melanoma skin cancer
- head, neck, esophagus
- pancreas cancer
- bladder cancer
- prostate cancer.
